(19) Europäisches Patentamt
European Patent Office
Office européen des brevets

(11) EP 1 533 415 B1

(12) **EUROPEAN PATENT SPECIFICATION**

(45) Date of publication and mention of the grant of the patent:
**14.02.2007 Bulletin 2007/07**

(21) Application number: **04257187.7**

(22) Date of filing: **19.11.2004**

(51) Int Cl.:
***D06M 13/00*** (2006.01) ***D06M 23/12*** (2006.01)
***C11D 3/50*** (2006.01) ***A61L 9/01*** (2006.01)
***A61L 9/14*** (2006.01) ***A61L 9/04*** (2006.01)

(54) **Melamine-formaldehyde microcapsule slurries for fabric article freshening**

Aufschlämmungen von Melamin-Formaldehyd-Mikrokapseln zur Textilauffrischung

Suspension de microcapsule de melamine-formaldehyde pour refraîchir des articles textiles

(84) Designated Contracting States:
**DE ES FR GB IT**

(30) Priority: **20.11.2003 US 718239**

(43) Date of publication of application:
**25.05.2005 Bulletin 2005/21**

(73) Proprietor: **INTERNATIONAL FLAVORS & FRAGRANCES INC.**
**New York**
**New York 10019 (US)**

(72) Inventors:
- **Parekh, Prabodh P.**
**Marlboro**
**New Jersey 07746 (US)**
- **Fernandez, Guillermo H.**
**Lebanon**
**New Jersey 08832 (US)**
- **Colt, Kristine K.**
**Jackson**
**New Jersey 08527 (US)**

(74) Representative: **Mercer, Christopher Paul et al**
**Carpmaels & Ransford,**
**43-45 Bloomsbury Square**
**London WC1A 2RA (GB)**

(56) References cited:
**EP-A- 0 328 937**
**WO-A-02/085420**
**WO-A-03/089561**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European Patent Convention).

EP 1 533 415 B1

## Description

### FIELD OF THE INVENTION

Freshening fabric articles by means of spraying the articles with aqueous slurry of microcapsules having rupturable melamine-formaldehyde polymeric walls, containing substantive and efficacious malodour counteractants and/or fragrances.

### BACKGROUND OF THE INVENTION

The need for application of various functional products, e.g., malodour counteractants to wearable fabrics with at least a modicum of permanency has been well-recognized throughout the history of modem mankind. Thus, fabrics containing imbedded microcapsules composed of melamine-formaldehyde polymeric shells containing fragrances are disclosed in the prior art, for example, U.S. Patent 4,917,920. In addition, processes for spraying fabric wrinkle control agents together with materials which remove or reduce undesirable odor from malodourous fabric are known in the prior art, for example, U.S. Patents 6,001,343 and 6,146,621. In addition, methods for spraying functional product-containing microcapsules, for example, fragrance-containing microcapsules, onto fabrics are disclosed in the prior art, U.S. Patent No. 6,071,569. Furthermore, methods for spraying microcapsules such as those composed of melamine-formaldehyde polymeric shells containing fragrances, including malodour-counteracting fragrances onto substrates such as floors are also suggested in the prior art, for example, PCT Published Patent WO 02/085420 A1. However, nothing set forth in the prior art either explicitly or implicitly describes a technique for the permanent deposition of effectively-rupturable malodour suppressant and/or fragrance emitting microcapsules onto fabrics wherein the resulting emitted fragrance activity and/or malodour counteractant activity is long-lasting and where the resulting substantive aroma is aesthetically pleasing over the long period of time for which it is effective.

U.S. Patent 2005 0106 192 filed on November 13, 2003 discloses the synergistic malodour counteractant composition of zinc ricinoleate and substituted monocyclic organic compounds such as 1-cyclohexylethan-1-yl butyrate but does not disclose the use of such compositions in microencapsulated form or in aqueous slurries.

### SUMMARY OF THE INVENTION

Our invention is directed to a process for freshening fabric articles by means of spraying the articles with an aqueous slurry of microcapsules each of which microcapsule has a rupturable melamine-formaldehyde polymeric wall, containing substantive and efficacious malodour counteractants and/or fragrances and to compositions of matter which include microencapsulated products useful in connection with the carrying out of the aforementioned process.

More specifically, our invention is directed to a method for substantively (a) imparting fragrance to and/or (b) substantially eliminating malodours from and/or (c) covering malodours evolved from and/or (d) preventing malodour formation in at least one fabric article for an extended period of time comprising the steps of:

i. providing one or more exposed surface areas of one or more fabric articles;

ii. providing an enclosure equipped with at least one pressure-activated air atomizer having an externally-located spray nozzle communicating with the interior of said enclosure, said nozzle having from 1 up to a plurality of nozzle exit ports each of which has a nozzle exit port effective diameter, $D_{NPi}$;

iii. preparing a plurality of microcapsules each of which is composed of a rupturable external wall of a melamine-formaldehyde polymer enclosing from about 10 weight % to about 30 weight % of a first functional substance which is one or more of (a) a fragrance composition each of the components of which has a C $\log_{10}P$ of between 2.5 and 8, wherein P is the n-octanol/water partition coefficient of said component; (b) a malodour counteracting composition each of the components of which has a C $\log_{10}P$ of between 2.5 and 8, wherein P is the n-octanol/water partition coefficient of said component; and/or (c) a malodour-preventing composition each of the components of which has a C $\log_{10}P$ of between 2.5 and 8, wherein P is the n-octanol/water partition coefficient of said component, each of said microcapsules having an average effective diameter $\overline{D_{MC}}$ of from about 5 microns to about 80 microns, each of which microcapsule has an effective diameter of $D_{MCi}$ wherein the smallest of $D_{NPi}$ is substantially greater than the greatest of $D_{MCi}$ wherein $\overline{D_{MC}}$ = and N represents the number of microcapsules in the slurry contained in said enclosure;

iv. providing an aqueous solution comprising (a) water, (b) a compatible solvent which is one or more of ethanol, the mono-$C_1$ or $C_2$ ether of a mono-, di-, or tri-1,2-propylene glycol and/or the di-$C_1$ or $C_2$ ether of a mono-, di- or tri-1,2-propylene glycol, (c) a compatible silicone polymer, (d) a compatible non-ionic surfactant, (e) a compatible preservative and (f) a compatible suspending agent;

v. admixing said plurality of microcapsules with said aqueous solution at a level of from about 0.1 weight % to about 0.4 weight % of microcapsules based on the weight of aqueous solution, thereby forming a microcapsule slurry wherein said microcapsules are suspended in said slurry and each of said microcapsules has a settling velocity in said slurry, $V_S$ equal to about 0;

vi. optionally causing a non-encapsulated second functional substance which is one or more of (a) a fragrance composition each of the components of which has a C $\log_{10}P$ of between 1 and 8, wherein P is the n-octanol/water partition coefficient of said component; (b) a malodour counteracting composition each of the components of which has a C $\log_{10}P$ of between 1 and 8, wherein P is the n-octanol/water partition coefficient of said component; and/or (c) a malodour-preventing composition each of the components of which has a C $\log_{10}P$ of between 1 and 8, wherein P is the n-octanol/water partition coefficient of said component to be in admixture with said slurry by means of admixing said second functional composition (A) with said aqueous solution and/or (B) with said slurry;

vii. placing said microcapsule slurry into said enclosure;

viii. situating said enclosure whereby the nozzle exit ports of the externally-located spray nozzle are each substantially located in a plane substantially parallel to and opposite said one or more exposed surface areas of said one or more fabric articles at a substantially perpendicular mean distance of from about 0 to about 3 meters from said one or more exposed surface areas of said one or more fabric articles;

ix. applying sufficient pressure to said slurry located within said enclosure to enable said slurry to be sprayed through said one or more nozzle exit ports onto said one or more exposed surface areas of said one or more fabric articles whereby said microcapsules are effectively adhered to said one or more exposed surface areas of said one or more fabric articles thereby forming one or more microcapsule-fixed fabric article surface areas; and

whereby (a) the concentration of the functional substance contained in the slurry is from about 0.03% to about 0.8% , preferably from about 0.05% to about 0.3% immediately prior to the step ix and (b) subsequent to the step ix when said microcapsule-fixed fabric article surface areas are rubbed, said microcapsules rupture, thereby emitting said first functional substance.

The aforementioned first functional substance and/or second functional substance may comprise a mixture of zinc ricinoleate or a solution thereof and a substituted monocyclic organic compound which is in the alternative or in combination one or more of:

1-cyclohexylethan-1-yl butyrate;

1-cyclohexylethan-1-yl acetate;

1-cyclohexylethan-1-ol;

1-(4'-methylethyl)cyclohexylethan-1-yl propionate; and

2'-hydroxy-1'-ethyl(2-phenoxy)acetate

each of which compound is marketed under the trademark VEILEX by International Flavors & Fragrances Inc., New York, N.Y., U.S.A.

Capsules having walls composed of a melamine-formaldehyde polymer containing such zinc ricinoleate-containing mixtures are novel compositions of matter.

Useful in the practice of our invention are microcapsules each of which is composed of a melamine-formaldehyde polymeric shell where there is enclosed within the shell a functional ingredient which is either (a) a fragrance composition each of the components of which has a C $\log_{10}P$ of between 2.5 and 8, wherein P is the n-octanol/water partition coeffiicent of said component, and/or (b) a malodour counteracting composition each of the components of which has a C $\log_{10}P$ of between 2.5 and 8, with the microcapsule being in intimate contact with one or more polymeric silicone phospholipids which is(are) included in the microcapsule slurry at a rate of 0.05% to 0.8%, preferably 0.1 - 0.3% and most preferably, 0.2%.

Preferably, such polymeric silicone phospholipid(s) is(are) prepared by the phosphation reaction of a terminal dimethicone copolyol with a phosphating agent followed by neutralization of the phosphate with base followed by a condensation reaction with an epihalohydrin followed by conducting a n-alkylation reaction with an amine.

In addition, all or a portion of the microcapsules useful in the practice of our invention may be coated with

coatings as described in Applications for U.S. Letters Patent Serial Nos. 10/268,566 and/or 10/268,526 each having been filed on October 10, 2002.

## DETAILED DESCRIPTION OF THE INVENTION

In carrying out the process of our invention, the enclosure which is equipped with a pressure-activated air atomizer is a spray dispenser which, most preferably is a manually or non-manually activated pump-spray dispenser for containing the slurry suspension to be applied to fabric articles in accordance with the process of our invention. A number of trigger sprayers or finger pump sprayers are suitable for carrying out the process of our invention, for example, the Indesco T-8500 sprayer available from Continental Sprayers, Inc. of St. Peters, Missouri, U.S.A. Other useful sprayers are described in U.S. Patent 4,082,223 and 4,819,835. Other useful sprayers for carrying out the process of our invention are disclosed in U.S. Patent 6,592,813.

Methods for preparing the functional product-encapsulates having melamine formaldehyde polymeric shells useful in the practice of our invention are disclosed in the prior art according to U.S. Patents 3,516,846; 4,681,806; 6,024,943; 6,194,375; 6,413,548; Published U.S. Patent Application 2003/0125222A1 and Published U.S. Patent Application 2003/0199412A1. The particle size range of the encapsulates useful in carrying out the process of our invention is in the range of 5 - 80 microns; preferably 5-40 microns; and most preferably 5-10 microns.

The functional product, e.g., fragrance and/or malodour counteractant encapsulated in the melamine-formaldehyde polymeric encapsulate used in the practice of our invention has a C $\log_{10}P$ of between 2.5 and 8, as stated above.

The values of C $\log_{10}P$ of many functional product ingredients, for example, fragrance ingredients contained in personal treatment compositions and/or cosmetic compositions is discussed in U.S. Patents 5,968,404 and 6,495,058. Furthermore, values of $\log_{10}P$ have been reported; for example, the Pomona92 database, available from Daylight Chemical Information Systems, Inc., Daylight CIS, Irvine, California. However, the $\log_{10}P$ values are most conveniently calculated by the "CLOGP" program, also available from Daylight CIS. This program also lists experimental $\log_{10}P$ values when they are available in the Pomona92 database. The "calculated $\log_{10}P$" (C $\log_{10}P$) is determined by the Hansch and Leo "fragment" approach based on the chemical structure of each functional product ingredient, and takes into account the numbers and types of atoms, the atom connectivity and the chemical bonding. The C $\log_{10}P$ values which are the most reliable and widely used estimates for this physicochemical property, are preferably used instead of the experimental $\log_{10}P$ values for the selection of functional ingredients, including perfume ingredients which are useful components in the microencapsulate-containing slurries of our invention.

Specific examples of preferred fragrance components useful in melamine-formaldehyde polymeric microencapsulates used in the process of our invention, and the molecular weights and $C\log_{10}P$ values of each of said components are set forth in Table I as follows:

**Table I**

| Fragance Component | $C\log_{10}P$ value | Molecular Weight |
|---|---|---|
| amyl salicylate | 4.601 | 208.26 |
| benzyl salicylate | 4.383 | 228.25 |
| β-caryophyllene | 6.333 | 204.36 |
| ethyl undecylenate | 4.888 | 212.34 |
| geranyl anthranilate | 4.216 | 273.38 |
| α-irone | 3.820 | 206.33 |
| β-phenyl ethyl benzoate | 4.058 | 226.28 |
| 1-phenyl hexanol-5 | 3.299 | 178.28 |
| α-santalol | 3.800 | 220.36 |
| amyl salicylate | 4.601 | 208.26 |
| β-caryophyllene | 6.333 | 204.36 |
| cedrol | 4.530 | 222.37 |
| cedryl acetate | 5.436 | 264.41 |
| cedryl formate | 5.070 | 238.37 |

(continued)

| Fragance Component | $Clog_{10}P$ value | Molecular Weight |
|---|---|---|
| cyclohexyl salicylate | 5.265 | 220.29 |
| γ-dodecalactone | 4.359 | 198.31 |
| β-phenylethyl phenyl acetate | 3.767 | 240.31 |
| 5-acetyl-1,1,2,3,3,6-hexamethyl indane | 5.977 | 258.41 |
| cyclopentadecanolide | 6.246 | 240.39 |
| d-limonene | 4.232 | 136.24 |
| amyl cinnamic aldehyde | 4.324 | 202.30 |
| linalyl benzoate | 5.233 | 258.36 |

The aqueous solution provided in step iv of the process of our invention is composed of components which do not detract from the desired effects of carrying out the process of our invention on fabric articles. For example, each components of such aqueous solution are to be substantially free of any substantially-detectable disagreeable aroma and no component is to leave any discemable residue subsequent to the drying of the product applied to the fabric article as a result of the carrying out of the process of our invention. Accordingly, in the process of our invention the aqueous solution provided in step iv, above, preferably consists essentially of (a) from about 80 to about 93 parts by weight of water; (b) from about 4 to about 8 parts by weight of ethanol or a methyl and/or ethyl ether of propylene glycol, dipropylene glycol and/or tripropylene glycol, with use of materials such as methyl cellosolve is contraindicated in view of the aroma thereof; (c) from about 2 to about 3 parts by weight of a compatible non-ionic surfactant; (d) from about 0.05 to about 0.5 parts by weight of a compatible preservative; (e) from about 0.1 to about 2 parts by weight of a compatible silicone polymer and (f) from about 0.05 to about 0.1 parts by weight of compatible suspending agent.

Preferred silicone polymers are emulsified silicones having the formula:

$$(CH_3)_3SiO[(CH_3)_2SiO]_mSi(CH_3)_2$$

wherein m is in the range of from 1 to 8. Other useful silicone polymers are polydimethyl siloxanes as disclosed in U.S. Patent 6,001,343 at Column 29, lines 1-25.

In the aqueous solution used in the process of our invention the non-ionic surfactant is preferred to be a mixture of the hydroxy-octaethoxy ethers of n-nonanol and n-undecanol (TOMADOL 91-8, trademark of Tomah Products, Inc.)., the compatible preservative is preferably hexahydro-1,3,5-tris(2-hydroxyethyl)-s-triazine (SURCIDE P available from Surety Laboratories) and the compatible suspending agent is preferably one or more of attapulgite clay, xanthan gum, hydroxypropyl cellulose having a molecular weight of from about 50,000 to about 800,000, colloidal silica and/or ethyl cellulose having a particle size of from about 0.004 microns to about 0.130 microns, a surface area of from about 100 $m^2$ per gram to about 500 $m^2$ per gram and a density of from about 1.0 to about 4.0 pounds per $ft^3$.

For purposes of substantivity enhancement of the delivery to the fabric article of the microencapsulated functional product in accordance with the process of our invention, the slurry containing the melamine-formaldehyde polymeric shells encapsulating the aforementioned fragrance and/or malodour suppressing or counteracting or preventing components may be in admixture with a silicone phospholipid polymer additive as exemplified in U.S. Patent 5,237,035, examples 56-85 at column 12 thereof. Such silicone phospholipid polymer additive(s) are included in the microcapsule slurry useful in the practice of our invention at a rate of 0.05% to 0.8%, preferably 0.1 - 0.3% and most preferably, 0.2%. Such slurries of containing the silicone phospholipid polymers are novel compositions of matter.

In addition, synergistic combinations of malodour counteractants may be included in the microencapsulated compositions useful in the practice of our invention, for example, the compositions comprising:

i. from about 10 to about 90 parts by weight of at least one substituted monocyclic organic compound-containing material which is:

(a) 1-cyclohexylethan-1-yl butyrate having the structure:

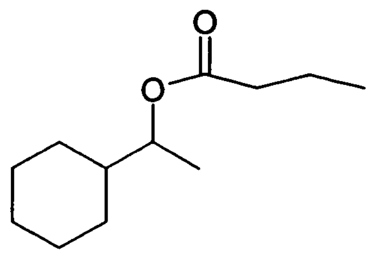

(b) 1-cyclohexylethan-1-yl acetate having the structure:

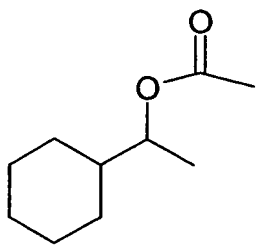

(c) 1-cyclohexylethan-1-ol having the structure:

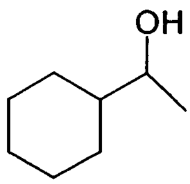

(d) 1-(4'-methylethyl)cyclohexylethan-1-yl propionate having the structure:

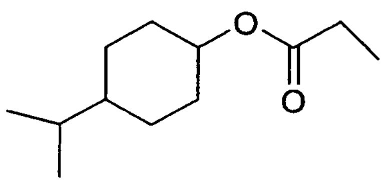

and

(e) 2'-hydroxy-1'-ethyl(2-phenoxy)acetate having the structure:

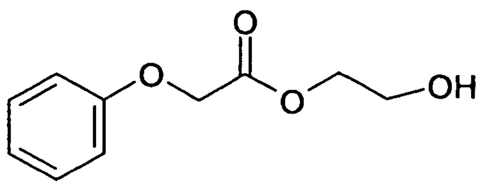

and

ii. from about 90 to about 10 parts by weight of a zinc ricinoleate-containing composition which is zinc ricinoleate

and/or solutions of zinc ricinoleate containing greater than about 30% by weight of zinc ricinoleate. Preferably, the aforementioned zinc ricinoleate-containing compositions are mixtures of about 50% by weight of zinc ricinoleate and about 50% by weight of at least one 1-hydroxy-2-ethoxyethyl ether of a $C_{12}$-$C_{14}$ fatty alcohol.

More specifically, a preferred composition useful in combination with the zinc ricinoleate component is a mixture of:

(A) 1-cyclohexylethan-1-yl butyrate;
(B) 1-cyclohexylethan-1-yl acetate; and
(C) 1-(4'-methylethyl)cyclohexylethan-1-yl propionate.

More preferably, the weight ratio of components of the immediately-aforementioned zinc riconoleate-containing mixture is one where the zinc ricinoleate-containing composition: 1-cyclohexylethan-1-yl butyrate:1-cyclohexylethan-1-yl acetate:1-(4'-methylethyl)-cyclohexylethan-1-yl propionate is about 2:1:1:1.

Another preferred composition useful in combination with the zinc ricinoleate component or solution is a mixture of:

(A) 1-cyclohexylethan-1-yl acetate; and
(B) 1-(4'-methylethyl)cyclohexylethan-1-yl propionate.

More preferably, the weight ratio of components of the immediately-aforementioned zinc riconoleate mixture is one where the zinc ricinoleate-containing composition: 1-cyclohexylethan-1-yl acetate:1-(4'-methylethyl)cyclohexylethan-1-yl propionate is about 3:1:1.

Optionally, the slurry used in practicing the process of our invention may also contain non-confined (or 'non-encapsulated") functional product, e.g., fragrance, each of the components of which has a C $\log_{10}P$ of between 1 and 8, for example, those set forth in the following Table II:

**Table II**

| Fragance Component | $C\log_{10}P$ value | Molecular Weight |
|---|---|---|
| benzaldehyde | 1.480 | 106.12 |
| benzyl acetate | 1.960 | 150.17 |
| laevo-carvone | 2.083 | 150.22 |
| geraniol | 2.649 | 154.26 |
| cis-jasmone | 2.712 | 164.25 |
| β-phenylethyl alcohol | 1.183 | 122.17 |
| α-terpineol | 2.569 | 154.25 |
| δ-nonalactone | 2.760 | 156.23 |
| 1-phenyl hexanol-5 | 3.299 | 178.28 |
| dihydromyrcenol | 3.03 | 156.27 |
| δ-undecalactone | 3.830 | 184.28 |
| amyl cinnamate | 3.771 | 218.30 |
| benzophenone | 3.120 | 182.22 |
| nerol | 2.649 | 154.25 |
| 2-methoxynaphthalene | 3.235 | 158.20 |
| ethyl undecylenate | 4.888 | 212.34 |
| geranyl anthranilate | 4.216 | 273.38 |
| α-irone | 3.820 | 206.33 |
| α-santalol | 3.800 | 220.36 |

(continued)

| Fragance Component | $Clog_{10}P$ value | Molecular Weight |
|---|---|---|
| iso-eugenol | 2.547 | 164.21 |
| amyl salicylate | 4.601 | 208.26 |
| benzyl salicylate | 4.383 | 228.25 |
| β-caryophyllene | 6.333 | 204.36 |
| cedrol | 4.530 | 222.37 |
| cedryl acetate | 5.436 | 264.41 |
| cedryl formate | 5.070 | 238.37 |
| cyclohexyl salicylate | 5.265 | 220.29 |
| γ-dodecalactone | 4.359 | 198.31 |
| ethyl undecylenate | 4.888 | 212.34 |
| geranyl anthranilate | 4.216 | 273.38 |
| β-phenylethyl benzoate | 4.058 | 226.38 |
| β-phenylethyl phenyl acetate | 3.767 | 240.31 |
| 5-acetyl-1,1,2,3,3,6-hexamethyl indane | 5.977 | 258.41 |
| cyclopentadecanolide | 6.246 | 240.39 |
| d-limonene | 4.232 | 136.24 |
| cis-p-t-butylcyclohexyl acetate | 4.019 | 198.31 |
| amyl cinnamic aldehyde | 4.324 | 202.30 |

It is to be understood that the preferred range of concentration of functional product contained in the slurry of our invention, both encapsulated and non-confined is from about 0.03 to about 0.8% by weight; preferably from about 0.05 to about 0.3 percent by weight of the slurry.

Subsequent to the slurry being applied to the fabric article according to the process of our invention, the algorithm of the scaled intensity of functional product evolved from the thus- treated article, Y, vs. time (in days), X is as follows:

$$Y = \alpha X^3 + \beta X^2 + \gamma X + \delta$$

wherein
$-0.05 \leq \alpha \leq +0.03$;
$-2.0 \leq \beta \leq +0.3$;
$-1.0 \leq \gamma \leq +5.0$; and
$-1.0 \leq \delta \leq +5.0$.

## BRIEF DESCRIPTION OF THE DRAWINGS

Figures 1, 2, 3 and 4 are sets of bar graphs for, respectively, 0, 3, 7 and 14 day evaluation periods comparing fragrance (of Example A described herein) intensity on a scale of 0-5 (measured on the "Y' axis) vs. time (measured on the "X" axis) for (a) fragrances evolving from several types of microcapsule compositions, for example, those containing microcapsules composed of melamine formaldehyde polymeric shells containing functional product, those composed of urea formaldehyde shells containing functional product, those composed of melamine-formaldehyde shells containing functional product with the slurry having added thereto silicone phospholipid polymers in a concentration of 0.2% and those composed of microcapsules formed by coacervation of a gelatin capsule around a liquid phase fragrance composition and (b) neat fragrance, each being deposited in an amount of 1 gram on circular areas of 1" radius of wearable fabric articles (cotton T-shirts) using the process of our invention both before rubbing and after rubbing. In all cases, the fragrance in the composition being evaluated is at a level of 0.05% by weight of the composition being evaluated. In all

cases each of the microcapsules and the neat fragrance is contained in a base consisting of water, ethanol, suspending agent, non-ionic surfactant, preservative and silicone polymer.

The evaluation at "0" time is herein intended to mean an evaluation taking place 3 hours subsequent to the composition application onto the fabric article, in order that the composition is dry at the time of evaluation.

Figure 5 is a set of 10 day stability bar graphs for an initial evaluation (3 hours after application) comparing, at room temperature 20°C (68°F) and at oven temperature 49°C (120°F), fragrance intensity on a scale of 0-5 (measured on the "Y' axis) vs. time (measured on the "X" axis) for (a) fragrances evolving from several types of microcapsules, such as those composed of melamine formaldehyde polymeric shells, those composed of urea formaldehyde shells, and those formed by coacervation of a gelatin capsule around a liquid phase fragrance composition (powdered and non-powdered) and (b) neat fragrance deposited on fabric swatches using the process of our invention both before rubbing and after rubbing. In all cases each of the microcapsules and the neat fragrance is contained in a base consisting of water, ethanol, suspending agent, non-ionic surfactant, preservative and silicone polymer. In all cases, the fragrance in the composition being evaluated is at a level of 0.05% by weight of the composition.

Figure 6 is a set of stability bar graphs for an initial head space analysis (via total area count in a gas chromatogram) and a 10 day head space analysis (via total area count in a gas chromatogram) comparing, at refrigerator temperature 33°C (38°F), at room temperature 20°C (68°F) and at oven temperature 49°C (120°F), headspace fragrance concentration as measured by gas chromatogram area count (on the "Y" axis) for a base consisting of water, ethanol, suspending agent, non-ionic surfactant, preservative and silicone polymer containing (a) no fragrance, (b) neat fragrance or (c) fragrance microencapsulated in microcapsules composed of melamine-formaldehyde polymeric shells deposited on fabric swatches using the process of our invention. Measurements are carried out before rubbing.

Figures 7A, 7B, 7C and 7D are sets of bar graphs for, respectively, 1, 2, 3 and 4 day evaluation periods comparing fragrance (of Example B, infra) intensity on a scale of 0-5 (measured on the "Y' axis) vs. time (measured on the "X" axis) for (a) fragrance evolving from microcapsules composed of melamine formaldehyde polymeric shells; (b) neat fragrance (of Example B, infra); (c) both fragrance evolving from microcapsules composed of melamine formaldehyde polymeric shells (50%) and neat fragrance (of Example B, infra) (50%); and (d) commercial base ( Stop and Shop, Inc. FABRIC EASE base) each deposited on a circular area of 1" radius of a fabric article (cotton T-shirts) in an amount of 1 gram using the process of our invention. Evaluations are carried out both before rubbing and after rubbing.

In all cases except for the one concerning the commercial base, each of the microcapsules, and the neat fragrance is contained in a base consisting of water, ethanol, suspending agent, non-ionic surfactant, preservative and silicone polymer.

In all cases, the fragrance in the composition being evaluated is at a level of 0.10% by weight of the composition being evaluated.

In all cases, the evaluations are made by a 10 member expert panel.

Figure 8 is a set of graphs of mathematical models for evaluations of scaled intensity vs. time (in days) for each of the results of Figures 1, 2, 3 and 4. The evaluations are those of non-rubbed and rubbed fabric articles (cotton T-shirts) having applied thereto in an amount of 1 gram on a circular area 1" in radius, slurries containing (a) melamine-formaldehyde polymeric fragrance-containing microcapsules and (b) slurries containing melamine-formaldehyde polymeric fragrance-containing microcapsules with 0.2% by weight of a silicone phospholipid polymer being added thereto. In each case the mathematical model is in accordance with the generic equation:

$$Y = \alpha X^3 + \beta X^2 + \gamma X + \delta$$

Figure 9 is a set of graphs of mathematical models for evaluations of scaled intensity vs. time (in days) for each of the results of Figures 7A, 7B, 7C and 7D. The evaluations are for non-rubbed and rubbed fabric articles (cotton T-shirts) having applied thereto in an amount of 1 gram on a circular area 1" in radius, slurries containing (a) melamine-formaldehyde polymeric fragrance containing microcapsules and (b) 50:50 mixtures of non-encapsulated fragrance and melamine-formaldehyde polymeric fragrance containing microcapsules. In each case the mathematical model is in accordance with the generic equation:

$$Y = \alpha X^3 + \beta X^2 + \gamma X + \delta$$

## DETAILED DESCRIPTION OF THE DRAWINGS

In Figures 1, 2, 3 and 4 the "X" axis, along which each of the bar graphs is placed, is indicated by reference

numeral 16, and the "Y" axis, representing scaled intensity of fragrance (on a scale of (0 - 5) observed after treatment is indicated by reference numeral 15. Figure 1 sets forth evaluations at 0 days (3 hours after application of the test substance to the fabric article). Figures 2, 3 and 4 respectively set forth evaluations at 3, 7 and 14 days. The bar graphs indicated by reference numerals 10A, 20A, 30A and 40A are for non-rubbed fabric articles (cotton T-shirts) having neat fragrance (of Example A infra) applied in an amount of 1 gram over a circular area of 1" in radius, and their evaluations at, respectively, 0, 3, 7 and 14 days. The bar graphs indicated by reference numerals 10B, 20B, 30B and 40B are for rubbed fabric articles (cotton T-shirts) having neat fragrance (of Example A infra) applied in an amount of 1 gram over a circular area of 1" in radius, and their evaluations at, respectively, 0, 3, 7 and 14 days. The bar graphs indicated by reference numerals 11A, 21A, 31A and 41A are for non-rubbed fabric articles (cotton T-shirts) having microcapsules composed of melamine-formaldehyde polymeric shells encapsulating fragrance (of Example A infra) applied in an amount of 1 gram over a circular area 1" in radius, and their evaluations at, respectively, 0, 3, 7 and 14 days. The bar graphs indicated by reference numerals 11B, 21B, 31B and 41B are for rubbed fabric articles (cotton T-shirts) having microcapsules composed of melamine-formaldehyde polymeric shells encapsulating fragrance (of Example A infra) applied in an amount of 1 gram over a circular area 1" in radius and their evaluations at, respectively, 0, 3, 7 and 14 days.

The bar graphs indicated by reference numerals 12A, 22A, 32A and 42A are for non-rubbed fabric articles (cotton T-shirts) having microcapsules composed of coacervated gelatin shells encapsulating fragrance (of Example A infra) applied in an amount of 1 gram over a circular area 1" in radius and their evaluations at, respectively, 0, 3, 7 and 14 days. The bar graphs indicated by reference numerals 12B, 22B, 32B and 42B are for rubbed fabric articles (cotton T-shirts) having microcapsules composed of coacervated gelatin shells encapsulating fragrance (of Example A infra) applied in an amount of 1 gram over a circular area 1" in radius and their evaluations at, respectively, 0, 3, 7 and 14 days. The coacervated gelatin microcapsules containing fragrances are prepared in accordance with the process described in U.S. Patent 2,800,457 and more particularly in Example D at Column 19, lines 51-65 of U.S. Patent 4,428,869. The bar graphs indicated by reference numerals 13A, 23A, 33A and 43A are for non-rubbed fabric articles (cotton T-shirts) having microcapsules composed of urea-formaldehyde polymeric shells encapsulating fragrance (of Example A infra) applied in an amount of 1 gram over a circular area 1" in radius and their evaluations at, respectively, 0, 3, 7 and 14 days. The fragrance-containing microcapsules composed of urea-formaldehyde shells are prepared in accordance with the procedure of Example 2 at Column 8, lines 28-47 of U.S. Patent 3,516,846. The bar graphs indicated by reference numerals 13B, 23B, 33B and 43B are for rubbed fabric articles 9cotton T-shirts) having microcapsules composed of urea-formaldehyde polymeric shells encapsulating fragrance (of Example A infra) applied in an amount of 1 gram over a circular area of 1" in radius and their evaluations at, respectively, 0, 3, 7 and 14 days. The bar graphs indicated by reference numerals 14A, 24A, 34A and 44A are for non-rubbed fabric articles (cotton T-shirts) having a slurry of fragrance (of Example A) containing microcapsules composed of melamine-formaldehyde polymeric shells in admixture with 0.2% by weight of added polymeric silicone phospholipid (prepared according to Example 56 at Column 12, line 7 of U.S. Patent 5,237,035) applied in an amount of 1 gram over a circular area of 1" in radius and their evaluations at, respectively, 0, 3, 7 and 14 days. The bar graphs indicated by reference numerals 14B, 24B, 34B and 44B are for rubbed fabric articles (cotton T-shirts) having microcapsules composed of melamine-formaldehyde polymeric shells in admixture with 0.2% by weight of added polymeric silicone phospholipid (prepared according to Example 56 at Column 12, line 7 of U.S. Patent 5,237,035) encapsulating fragrance (of Example A infra) applied in an amount of 1 gram over a circular area of 1" in radius and their evaluations at, respectively, 0, 3, 7 and 14 days.

The following table sets forth the weight percent fragrance, weight percent fragrance containing microcapsules, and weight percent fragrance-free microcapsules contained in the application slurries used in compiling the data for Figures 1, 2, 3 and 4, described above:

**Table III**

| Bar Graph Group | % Fragrance in Slurry | % microcapsule containing fragrance in slurry | % microcapsule (in absence of fragrance) in slurry |
|---|---|---|---|
| A | 0.05 | 0.00 | 0.00 |
| B | 0.05 | 0.18 | 0.13 |
| C | 0.05 | 0.21 | 0.16 |
| D | 0.05 | 0.07 | 0.02 |
| E | 0.05 | 0.38 | 0.33 |

In Figure 5 showing the results of 10 day stability studies, the "X" axis, along which each of the bar graphs is placed, is indicated by reference numeral 560, and the "Y" axis, representing scaled intensity of fragrance (on a scale

of 0 - 5) observed after treatment is indicated by reference numeral 550. The bar graphs indicated by reference numerals 50A and 51A are for the evaluations of non-rubbed fabric swatches maintained, respectively, at 49°C (120°F) and 20°C (68°F), having neat fragrance (of Example A infra) applied. The bar graphs indicated by reference numerals 50B and 51B are for the evaluations of rubbed fabric swatches maintained, respectively at 120°F and 68°F, having neat fragrance (of Example A infra) applied. The bar graphs indicated by reference numerals 52A and 53A are for the evaluations of non-rubbed fabric swatches maintained, respectively, at 49°C (120°F) and 20°C (68°F), having microcapsules composed of melamine-formaldehyde polymeric shells encapsulating fragrance (of Example A infra) applied. The bar graphs indicated by reference numerals 52B and 53B are for the evaluations of rubbed fabric swatches, maintained, respectively at 120°F and 68°F, having microcapsules composed of melamine-formaldehyde polymeric shells encapsulating fragrance (of Example A infra) applied. The bar graphs indicated by reference numerals 54A and 55A are for the evaluations of non-rubbed fabric swatches, maintained respectively at 49°C (120°F) and 20°C (68°F), having microcapsules composed of coacervated gelatin shells encapsulating fragrance (of Example A infra) applied. The bar graphs indicated by reference numerals 54B and 55B are for the evaluations of rubbed fabric swatches, maintained respectively at 120°F and 68°F, having microcapsules composed of coacervated gelatin shells encapsulating fragrance (of Example A infra) applied. The coacervated gelatin microcapsules containing fragrances are prepared in accordance with the process described in U.S. Patent 2,800,457 and more particularly in Example D at Column 19, lines 51-65 of U.S. Patent 4,428,869. The bar graphs indicated by reference numerals 56A and 57A are for the evaluations of non-rubbed fabric swatches, maintained respectively at 49°C (120°F) and 20°C (68°F), having microcapsules composed of urea-formaldehyde polymeric shells encapsulating fragrance (of Example A infra) applied. The bar graphs indicated by reference numerals 56B and 57B are for the evaluations of rubbed fabric swatches, maintained respectively at 120°F and 68°F, having microcapsules composed of urea-formaldehyde polymeric shells encapsulating fragrance (of Example A infra) applied. The bar graphs indicated by reference numerals 58A and 59A are for the evaluations of non-rubbed fabric swatches, maintained respectively at 120°F and 68°F, having powdered microcapsules composed of coacervated gelatin shells encapsulating fragrance (of Example A infra) applied. The bar graphs indicated by reference numerals 58B and 59B are for the evaluations of rubbed fabric swatches, maintained respectively at 49°C (120°F) and 20°C (68°F), having powdered microcapsules composed of coacervated gelatin shells encapsulating fragrance (of Example A infra) applied.

In all cases, the fragrance in the composition being evaluated is at a level of 0.05% by weight of the composition being evaluated.

In Figure 6, the set of stability bar graphs, total area count is measured on the "Y" axis, indicated by reference numeral 65 and the bar graphs are indicated along the "X" axis, indicated by reference numeral 66. The bar graphs indicated by reference numerals 61A, 62A, 63A and 64A are, respectively, for the measurement of the stability of neat fragrance (of Example A infra) at a level of 0.1% in base containing water, ethanol, suspending agent, non-ionic surfactant, preservative and silicone polymer for 0 days, 10 days at refrigeration temperature (38°F), 10 days at room temperature (68°F) and 10 days at oven temperature (120°F). The bar graphs indicated by reference numerals 61B, 62B, 63B and 64B are, respectively, for the measurement of the stability of microcapsules composed of melamine-formaldehyde polymeric shells encapsulating fragrance (of Example A infra) at a fragrance level of 0.1% in base containing water, ethanol, suspending agent, non-ionic surfactant, preservative and silicone polymer for 0 days, 10 days at refrigeration temperature 33°C (38°F), 10 days at room temperature 20°C (68°F) and 10 days at oven temperature 49°C (120°F). The bar graphs indicated by reference numerals 62C, 63C and 64C are, respectively, for the measurement of the stability of microcapsules composed of empty melamine-formaldehyde polymeric shells (not containing any fragrance or other functional product) for 10 days at refrigeration temperature (38°F), 10 days at room temperature 20°C (68°F) and 10 days at oven temperature 49°C (120°F). The weight % microcapsule containing fragrance in the slurry is 0.27% and the weight % empty microcapsule in the slurry is 0.17%. The results shown in Figure 6 are indicative of the high degree of stability of fragrances encapsulated in microcapsules composed of melamine-formaldehyde polymeric shells.

In Figures 7A, 7B, 7C and 7D the "X" axis, along which each of the bar graphs is placed, is indicated by reference numeral 702, and the "Y" axis, representing scaled intensity of fragrance (on a scale of (0 - 5) observed after treatment is indicated by reference numeral 701.

In Figure 7A, showing test results at 24 hours (1 day) after application, the bar graphs indicated by reference numerals 71A, 72A, 73A and 74A represent the evaluations of non-rubbed fabric articles (cotton T-shirts) respectively having applied thereto in an amount of 1 gram over a circular area 1" in radius (a) neat fragrance (of Example B, infra) contained at a level of 0.1% in a base consisting of water, ethanol, suspending agent, non-ionic surfactant, preservative and silicone polymer; (b) a composition consisting of 50% microencapsulated fragrance (of Example B, infra) contained in microcapsules composed of melamine-formaldehyde shells wherein the fragrance is contained at a level of 0.05% in a base consisting of water, ethanol, suspending agent, non-ionic surfactant, preservative and silicone polymer and 50% unconfined ("non-encapsulated") fragrance (of Example B, infra) at a level of 0.05% in a base consisting of water, ethanol, suspending agent, non-ionic surfactant, preservative and silicone polymer; (c) a composition consisting of microencapsulated fragrance (of Example B, infra) contained in microcapsules composed of melamine-formaldehyde shells wherein the fragrance is contained at a level of 0.10% in a base consisting of water, ethanol, suspending agent, non-ionic

surfactant, preservative and silicone polymer and (d) a commercial base ( Stop and Shop, Inc. FABRIC EASE base); and the bar graphs indicated by reference numerals 71B, 72B, 73B and 74B represent evaluations of rubbed fabric articles (cotton T-shirts) respectively having applied thereto in an amount of 1 gram over a circular area 1" in radius (a) neat fragrance (of Example B, infra) contained at a level of 0.1% in a base consisting of water, ethanol, suspending agent, non-ionic surfactant, preservative and silicone polymer; (b) a composition consisting of 50% microencapsulated fragrance (of Example B, infra) contained in microcapsules composed of melamine-formaldehyde shells wherein the fragrance is contained at a level of 0.05% in a base containing water, ethanol, suspending agent, non-ionic surfactant, preservative and silicone polymer and 50% non-encapsulated fragrance (of Example B, infra) at a level of 0.05% in a base containing water, ethanol, suspending agent, non-ionic surfactant, preservative and silicone polymer; (c) a composition consisting of microencapsulated fragrance (of Example B, infra) contained in microcapsules composed of melamine-formaldehyde shells wherein the fragrance is contained at a level of 0.10% in a base consisting of water, ethanol, suspending agent, non-ionic surfactant, preservative and silicone polymer and (d) a commercial base ( Stop and Shop, Inc. FABRIC EASE base).

In Figure 7B, showing test results at 48 hours (2 days) after application, the bar graphs indicated by reference numerals 81A, 82A, 83A and 84A represent evaluations of non-rubbed fabric articles (cotton T-shirts) respectively having applied thereto in an amount of 1 gram over a circular area 1" in radius (a) neat fragrance (of Example B, infra) contained at a level of 0.1% in a base consisting of water, ethanol, suspending agent, non-ionic surfactant, preservative and silicone polymer; (b) a composition consisting of 50% microencapsulated fragrance (of Example B, infra) contained in microcapsules composed of melamine-formaldehyde shells wherein the fragrance is contained at a level of 0.05% in a base consisting of water, ethanol, suspending agent, non-ionic surfactant, preservative and silicone polymer and 50% unconfined ("non-encapsulated") fragrance (of Example B, infra) at a level of 0.05% in a base consisting of water, ethanol, suspending agent, non-ionic surfactant, preservative and silicone polymer; (c) a composition consisting of microencapsulated fragrance (of Example B, infra) contained in microcapsules composed of melamine-formaldehyde shells wherein the fragrance is contained at a level of 0.10% in a base consisting of water, ethanol, suspending agent, non-ionic surfactant, preservative and silicone polymer and (d) a commercial base ( Stop and Shop, Inc. FABRIC EASE base); and the bar graphs indicated by reference numerals 81B, 82B, 83B and 84B represent evaluations of rubbed fabric articles (cotton T-shirts) respectively having applied thereto in an amount of 1 gram over a circular area 1" in radius (a) neat fragrance (of Example B, infra) contained at a level of 0.1% in a base containing water, ethanol, suspending agent, non-ionic surfactant, preservative and silicone polymer; (b) a composition consisting of 50% microencapsulated fragrance (of Example B, infra) contained in microcapsules composed of melamine-formaldehyde shells wherein the fragrance is contained at a level of 0.05% in a base containing water, ethanol, suspending agent, non-ionic surfactant, preservative and silicone polymer and 50% non-encapsulated fragrance (of Example B, infra) at a level of 0.05% in a base containing water, ethanol, suspending agent, non-ionic surfactant, preservative and silicone polymer; (c) a composition consisting of microencapsulated fragrance (of Example B, infra) contained in microcapsules composed of melamine-formaldehyde shells wherein the fragrance is contained at a level of 0.10% in a base containing water, ethanol, suspending agent, non-ionic surfactant, preservative and silicone polymer and (d) a commercial base ( Stop and Shop, Inc. FABRIC EASE base).

In Figure 7C, showing test results at 72 hours (3 days) after application, the bar graphs indicated by reference numerals 91A, 92A, 93A and 94A represent evaluations of non-rubbed fabric articles (cotton T-shirts) respectively having applied thereto in an amount of 1 gram over a circular area of 1" in radius (a) neat fragrance (of Example B, infra) contained at a level of 0.1% in a base consisting of water, ethanol, suspending agent, non-ionic surfactant, preservative and silicone polymer; (b) a composition consisting of 50% microencapsulated fragrance (of Example B, infra) contained in microcapsules composed of melamine-formaldehyde shells wherein the fragrance is contained at a level of 0.05% in a base consisting of water, ethanol, suspending agent, non-ionic surfactant, preservative and silicone polymer and 50% non-encapsulated fragrance (of Example B, infra) at a level of 0.05% in a base consisting of water, ethanol, suspending agent, non-ionic surfactant, preservative and silicone polymer; (c) a composition consisting of microencapsulated fragrance (of Example B, infra) contained in microcapsules composed of melamine-formaldehyde shells wherein the fragrance is contained at a level of 0.10% in a base consisting of water, ethanol, suspending agent, non-ionic surfactant, preservative and silicone polymer and (d) a commercial base ( Stop and Shop, Inc. FABRIC EASE base); and the bar graphs indicated by reference numerals 91B, 92B, 93B and 94B represent evaluations of rubbed fabric articles (cotton T-shirts) respectively having applied thereto in an amount of 1 gram over a circular area 1" in radius (a) neat fragrance (of Example B, infra) contained at a level of 0.1% in a base consisting of water, ethanol, suspending agent, non-ionic surfactant, preservative and silicone polymer; (b) a composition consisting of 50% microencapsulated fragrance (of Example B, infra) contained in microcapsules composed of melamine-formaldehyde shells wherein the fragrance is contained at a level of 0.05% in a base consisting of water, ethanol, suspending agent, non-ionic surfactant, preservative and silicone polymer and 50% non-encapsulated fragrance (of Example B, infra) at a level of 0.05% in a base consisting of water, ethanol, suspending agent, non-ionic surfactant, preservative and silicone polymer; (c) a composition consisting of microencapsulated fragrance (of Example B, infra) contained in microcapsules composed of melamine-formaldehyde

shells wherein the fragrance is contained at a level of 0.10% in a base consisting of water, ethanol, suspending agent, non-ionic surfactant, preservative and silicone polymer and (d) a commercial base ( Stop and Shop, Inc. FABRIC EASE base).

In Figure 7D, showing test results at 96 hours (4 days) after application, the bar graphs indicated by reference numerals 101A, 102A, 103A and 104A represent evaluations of non-rubbed fabric articles (cotton T-shirts) respectively having applied thereto in an amount of 1 gram over a circular area 1" in radius (a) neat fragrance (of Example B, infra) contained at a level of 0.1% in a base consisting of water, ethanol, suspending agent, non-ionic surfactant, preservative and silicone polymer; (b) a composition consisting of 50% microencapsulated fragrance (of Example B, infra) contained in microcapsules composed of melamine-formaldehyde shells wherein the fragrance is contained at a level of 0.05% in a base consisting of water, ethanol, suspending agent, non-ionic surfactant, preservative and silicone polymer consisting of water, ethanol, suspending agent, non-ionic surfactant, preservative and silicone polymer; (c) a composition consisting of microencapsulated fragrance (of Example B, infra) contained in microcapsules composed of melamine-formaldehyde shells wherein the fragrance is contained at a level of 0.10% in a base consisting of water, ethanol, suspending agent, non-ionic surfactant, preservative and silicone polymer and (d) a commercial base ( Stop and Shop, Inc. FABRIC EASE base); and the bar graphs indicated by reference numerals 101B, 102B, 103B and 104B represent evaluations of rubbed fabric articles (cotton T-shirts) respectively having applied thereto in an amount of 1 gram over a circular area 1" in radius (a) neat fragrance (of Example B, infra) contained at a level of 0.1% in a base consisting of water, ethanol, suspending agent, non-ionic surfactant, preservative and silicone polymer; (b) a composition consisting of 50% microencapsulated fragrance (of Example B, infra) contained in microcapsules composed of melamine-formaldehyde shells wherein the fragrance is contained at a level of 0.05% in a base consisting of water, ethanol, suspending agent, non-ionic surfactant, preservative and silicone polymer and 50% non-encapsulated fragrance (of Example B, infra) at a level of 0.05% in a base consisting of water, ethanol, suspending agent, non-ionic surfactant, preservative and silicone polymer; (c) a composition consisting of microencapsulated fragrance (of Example B, infra) contained in microcapsules composed of melamine-formaldehyde shells wherein the fragrance is contained at a level of 0.10% in a base consisting of water, ethanol, suspending agent, non-ionic surfactant, preservative and silicone polymer and (d) a commercial base ( Stop and Shop, Inc. FABRIC EASE base).

Figure 8 shows a series of graphs of scaled intensity (on a scale of 0 - 5) vs. time (days) for the data shown in bar graphs 11A, 11B, 14A and 14B in Figure 1; bar graphs 21A, 21B, 24A and 24B in Figure 2; bar graphs 31A, 31B, 34A and 34B in Figure 3 and bar graphs 41A, 41B, 44A and 44B in Figure 4. Each of the graphs is a third degree equation. The scaled intensity is measured on the "Y" axis which is indicated by reference numeral 180. The time (in days) is measured along the "X" axis which is indicated by reference numeral 181. The following table sets forth the equation for each graph, the reference numeral indicating the graph, and the reference numerals identifying the specific bar graphs on which each graph is based:

**Table IV**

| Rubbed or unrubbed fabric article and substance applied thereto | Reference Numerals Identifying Relevant Bar Graphs | Equation | Reference Numeral for graph |
|---|---|---|---|
| Fragrances micro-encapsulated in melamine-formaldehyde polymeric microcapsules (non-rubbed) | 11A, 21A, 31A and 41A | $Y= -.0076X^3+0.157X^2 -0.770X+3.7$ | 182 |
| Fragrances micro-encapsulated in melamine-formaldehyde polymeric microcapsules (rubbed) | 11B, 21B, 31B and 41B | $Y= -.0058X^3+0.117X^2 -0.567X+4.5$ | 183 |
| Fragrances micro-encapsulated in melamine-formaldehyde polymeric microcapsules which are in admixture with polymeric silicone phospholipid additive (non-rubbed) | 14A, 24A, 34A and 44A | $Y= -.0036X^3+0.069X^2 -0.309X+3.2$ | 184 |

(continued)

| Rubbed or unrubbed fabric article and substance applied thereto | Reference Numerals Identifying Relevant Bar Graphs | Equation | Reference Numeral for graph |
|---|---|---|---|
| Fragrances micro-encapsulated in melamine-formaldehyde polymeric microcapsules which are in admixture with polymeric silicone phospholipid additive (rubbed) | 14B, 24B, 34B and 44B | $Y= -.0043X^3+0.084X^2 -0.381X+4.5$ | 185 |

Figure 9 shows a series of graphs of scaled intensity (on a scale of 0 - 5) vs. time (days) for the data shown in bar graphs 72A, 72B, 73A and 73B in Figure 7A; bar graphs 82A, 82B, 83A and 83B in Figure 7B; bar graphs 92A, 92B, 93A and 93B in Figure 7C and bar graphs 102A, 102B, 103A and 103B in Figure 7D. Each of the graphs is a third degree equation. The scaled intensity is measured on the "Y" axis which is indicated by reference numeral 190. The time (in days) is measured along the "X" axis which is indicated by reference numeral 191. The following table sets forth the equation for each graph, the reference numeral indicating the graph, and the reference numerals identifying the specific bar graphs on which each graph is based:

**Table V**

| Rubbed or unrubbed fabric article and substance applied thereto | Reference Numerals Identifying Relevant Bar Graphs | Equation | Reference Numeral for graph |
|---|---|---|---|
| 50:50 Mixture of fragrances+ fragrances microencapsulated in melamine-formaldehyde polymeric microcapsules (non-rubbed) | 72A, 82A, 92A and 102A | $Y= 0.233X^3-1.95X^2 +4.197X-0.5$ | 192 |
| 50:50 Mixture of fragrances+ fragrances microencapsulated in melamine-formaldehyde polymeric microcapsules (rubbed) | 72B, 82B, 92B and 102B | $Y= 0.133X^3-0.95X^2 +2.017X+3.0$ | 183 |
| Fragrances micro-encapsulated in melamine-formaldehyde polymeric microcapsules (non-rubbed) | 73A, 83A, 93A and 93A | $Y= -.05X^3+0.25X^2 -0.3X+4.3$ | 194 |
| Fragrances micro-encapsulated in melamine-formaldehyde polymeric microcapsules (rubbed) | 73B, 83B, 93B and 103B | $Y= 0.05X^3-0.25X^2 +0.3X+4.3$ | 195 |

The following examples are not meant to define or otherwise limit the scope of the invention. Rather the scope of the invention is to be ascertained according to the claims that follow the examples. Unless noted to the contrary, all percentages are given on a weight percent on a dry basis.

**Example A**

The following fragrance composition was prepared:

| Fragrance Component | C $\log_{10}P$ value | Molecular Weight | Parts by Weight |
|---|---|---|---|
| ethyl undecylenate | 4.888 | 212.34 | 3.0 |
| geranyl anthranilate | 4.216 | 273.38 | 7.5 |
| α-irone | 3.820 | 206.33 | 6.3 |
| phenyl ethyl benzoate | 4.058 | 226.28 | 3.2 |
| d-limonene | 4.232 | 136.24 | 3.2 |
| cis-p-t-butylcyclohexyl acetate | 4.019 | 198.31 | 5.8 |
| amyl cinnamic aldehyde | 4.324 | 202.30 | 7.3 |
| hexyl cinnamic aldehyde | 5.473 | 216.33 | 12.6 |
| hexyl salicylate | 5.260 | 222.29 | 12.6 |

**Example B**

The following fragrance was prepared:

| Fragrance Component | C $\log_{10}P$ value | Molecular Weight | Parts by Weight |
|---|---|---|---|
| ethyl undecylenate | 4.888 | 212.34 | 10.5 |
| geranyl anthranilate | 4.216 | 273.38 | 35.4 |
| α-irone | 3.820 | 206.33 | 5.3 |
| phenyl ethyl benzoate | 4.058 | 226.28 | 5.3 |
| phenylethyl phenyl acetate | 3.767 | 240.31 | 5.3 |
| 5-acetyl-1,1,2,3,3,6-hexamethyl indane | 5.977 | 258.41 | 2.5 |
| cyclopentadecanolide | 6.246 | 240.39 | 7.5 |
| d-limonene | 4.232 | 136.24 | 25.0 |
| cis-p-t-butylcyclohexyl acetate | 4.019 | 198.31 | 4.0 |
| amyl cinnamic aldehyde | 4.324 | 202.30 | 4.0 |

**EXAMPLE I**

At the following rate the fragrance of Example A was microencapsulated into the following microcapsules in accordance with the processes stated:

**Table VI**

| Nature of Capsule | Reference to Process for formation of microencapsulated functional product | % of functional product of Example A in microcapsule | Sample Identification |
|---|---|---|---|
| Melamine-formaldehyde polymer shell having average effective diameter of 5-10 microns | U.S.Patents 3,516,846 and 6,413,548 | 27.8% | B |
| Coacervated gelatin shell having average effective diameter of 10-20 microns | U.S. Patent 2,800,457 | 23.8% | C |

(continued)

| Nature of Capsule | Reference to Process for formation of microencapsulated functional product | % of functional product of Example A in microcapsule | Sample Identification |
|---|---|---|---|
| Urea-formaldehyde polymer shell having average effective diameter of 5-10 microns | U.S.Patent 3,516,846 | 71.4% | D |
| Melamine-formaldehyde polymer shell having average effective diameter of 5-10 microns coated with 53% silicone phospholipid polymer | U.S.. Patents 3,516,846; 6,413,548 and 5,237,035 | 37.6% | E |

A sufficient amount of each of the microcapsule products in the above table was placed in the following base:

(a) water- 90.7%
(b) ethanol- 5%
(c) SURCIDE P (Preservative)-0.1%
(d) TOMADOL 91-8 (non-ionic surfactant)- 2.7%
(e) Xanthan Gum- 0.5%
(f) dimethyl silicone polymer- 1.0%

in order to cause the functional product to be 0.05% of the slurry in each case.

In each case, the microencapsulated functional product was suspended as a "suspended slurry". 150 cubic centermeters of the resulting suspended slurry was then placed in a trigger sprayer as disclosed in U.S. Patent 4,819,835. In addition, neat fragrance of Example A (non-confined) was placed in a fifth sample of the above-identified base at the rate of 0.05% (identified as sample A).

In an individual amount of 1 gram, each of the resulting products was then simultaneously sprayed onto circular areas, 1 inch in radius of five separate equally malodourous cotton T-shirts having identical tobacco malodours.

The results of the spraying are set forth on Figures 1, 2, 3, and 4 described above. The numerical results for scaled intensity on a scale of 0-5 are set forth in the following Table VII:

**Table VII**

| Sample Identification | 0 DAYS | | 3 DAYS | | 7 DAYS | | 14 DAYS | |
|---|---|---|---|---|---|---|---|---|
| | not rubbed | rubbed | not rubbed | rubbed | not rubbed | rubbed | not rubbed | rubbed |
| A | 2.7 | 3 | 1.7 | 2.1 | 2.3 | 2.5 | 1.8 | 2.2 |
| B | 3.7 | 4.5 | 2.6 | 3.7 | 3.4 | 4.3 | 2.8 | 3.7 |
| C | 1.8 | 2.4 | 1.6 | 2.3 | 1.6 | 2.0 | 1.6 | 1.8 |
| D | 1.6 | 2.1 | 1.7 | 2.2 | 1.4 | 2.0 | 1.5 | 1.8 |
| E | 3.2 | 4.5 | 2.8 | 4.0 | 3.2 | 4.5 | 2.6 | 4.0 |

All evaluations leading to the results in Table VII were carried out by a 10 member expert panel.

The use of samples B and E, which both contained melamine-formaldehyde microencapsulated functional product covered the tobacco malodour in a manner far superior to that of samples A, C and D. In addition, the intensity of aesthetically-pleasing aroma prior to and after rubbing was significantly greater when using samples B and E than that of any aesthetically-pleasing aromas generated as a result of using any of samples A, C or D thereby showing the clear superiority of slurries containing microencapsulated functional products where the microcapsules are composed

of melamine-formaldehyde polymeric shells.

**EXAMPLE II**

At the rate of 28% the fragrance of Example B was microencapsulated in microcapsules produced according to U.S.Patents 3,516,846 and 6,413,548 and composed of melamine-formaldehyde polymer shells each of which had an average effective diameter of 5-10 microns.

A base was formulated containing the following ingredients:

(a) water - 90.7%
(b) ethanol - 5%
(c) SURCIDE P (Preservative) -0.1%
(d) TOMADOL 91-8 (non-ionic surfactant) - 2.7%
(e) Xanthan Gum - 0.5%
(f) dimethyl silicone polymer - 1.0%

A first group of microcapsules was formed into a slurry with the above-mentioned base whereby the resulting slurry contained 0.1% by weight of fragrance and the weight % of microcapsules containing fragrance was 0.27%. The slurry was identified by the letter "γ".

A second group of microcapsules was formed into a slurry with the above-mentioned base, in combination with non-confined fragrance of Example B whereby the resulting slurry contained 0.05% by weight of encapsulated fragrance and 0.05% by weight of non-confined ("non-encapsulated") fragrance and the weight % of microcapsules containing fragrance was 0.135%. The slurry was identified by the letter "β".

Non-confined fragrance of Example B as admixed with the above-mentioned base whereby the resulting formulation contained 0.1% by weight of fragrance. The resulting composition was identified by the letter "α".

A fourth sample was a commercial base, FABRIC EASE, marketed by Stop & Shop, Inc. The commercial base was identified by the letter, "δ".

Each of the compositions, "α", "β", "γ" and "δ" was applied via spraying in amounts of 1 gram each, separately, onto separate malodourous fabric articles (cotton T-shirts) emitting equal tobacco malodours, in circular areas having 1"radii.

The results of the spraying are set forth in Figures 7A, 7B, 7C and 7d described herein. The numerical results for scaled intensity on a scale of 0-5 are set forth in the following Table VIII:

**Table VIII**

| Sample Identification | 1 DAY | | 2 DAYS | | 3 DAYS | | 4 DAYS | |
|---|---|---|---|---|---|---|---|---|
| | not rubbed | rubbed | not rubbed | rubbed | not rubbed | rubbed | not rubbed | rubbed |
| α | 3.0 | 3.1 | 3.0 | 3.45 | 2.45 | 3.1 | 1.35 | 1.7 |
| β | 2.7 | 4.2 | 3.4 | 4.3 | 3.0 | 4.1 | 2.9 | 4.4 |
| γ | 2.9 | 4.4 | 3.0 | 4.3 | 3.0 | 4.3 | 2.6 | 4.7 |
| δ | 2.5 | 2.6 | 2.15 | 2.35 | 2.50 | 2.55 | 2.40 | 2.45 |

All evaluations leading to the results in Table VIII were carried out by a 10 member expert panel.

The use of samples "β" and "γ"which both contained melamine-formaldehyde microencapsulated functional product covered the tobacco malodour in a manner far superior to that of samples "α" and "δ". In addition, the intensity of aesthetically-pleasing aroma prior to and after rubbing was significantly greater when using samples "β" and "γ" than that of any aesthetically-pleasing aromas generated as a result of using any of samples "α" or "δ" thereby showing the clear superiority of slurries containing microencapsulated functional products where the microcapsules are composed of melamine-formaldehyde polymeric shells over bases containing non-confined fragrances.

## Claims

1. A method for substantively (a) imparting fragrance to and/or (b) substantially eliminating malodours from and/or (c) covering malodours evolved from and/or (d) preventing malodour formation in at least one fabric article for an extended period of time comprising the steps of:

i. providing one or more exposed surface areas of one or more fabric articles;
ii. providing an enclosure equipped with at least one pressure-activated air atomizer having an externally-located spray nozzle communicating with the interior of said enclosure, said nozzle having from 1 up to a plurality of nozzle exit ports having nozzle exit port effective diameters, $D_{NPi}$;
iii. preparing a plurality of microcapsules each of which is composed of a rupturable external wall of a melamine-formaldehyde polymer enclosing from about 10 weight % to about 30 weight % of a first functional substance selected from the group consisting of (a) a fragrance composition each of the components of which has a C $\log_{10}P$ of between 2.5 and 8, wherein P is the n-octanol/water partition coefficient of said component; (b) a malodour counteracting composition each of the components of which has a C $\log_{10}P$ of between 2.5 and 8, wherein P is the n-octanol/water partition coefficient of said component; and (c) a malodour-preventing composition each of the components of which has a C $\log_{10}P$ of between 2.5 and 8, wherein P is the n-octanol/water partition coefficient of said component, each of said microcapsules having an average effective diameter $\overline{D_{MC}}$ of from about 5 microns to about 80 microns, each of which microcapsule has an effective diameter of $D_{MCi}$ wherein the smallest of $D_{NPi}$ is substantially greater than the greatest of $D_{MCi}$ wherein $\overline{D_{MC}} = \frac{1}{N}\sum D_{MCi}$ and N represents the number of microcapsules in the slurry contained in said enclosure;
iv. providing an aqueous solution comprising (a) water, (b) a compatible solvent selected from the group consisting of ethanol, the mono-$C_1$ or $C_2$ ether of a mono-, di-, or tri-1,2-propylene glycol and the di-$C_1$ or $C_2$ ether of a mono-, di- or tri-1,2-propylene glycol, (c) a silicone polymer, (d) a compatible non-ionic surfactant, (e) a compatible preservative and (f) a compatible suspending agent;
v. admixing said plurality of microcapsules with said aqueous solution at a level of from about 0.1 weight % to about 0.4 weight % of microcapsules based o the weight of aqueous solution, thereby forming a microcapsule slurry wherein said microcapsules are suspended in said slurry and each of said microcapsules has a settling velocity in said slurry, $V_S$ equal to about 0;
vi. optionally causing a non-encapsulated second functional substance selected from the group consisting of (a) a fragrance composition each of the components of which has a C $\log_{10}P$ of between 1 and 8, wherein P is the n-octanol/water partition coefficient of said component; (b) a malodour counteracting composition each of the components of which has a C $\log_{10}P$ of between 1 and 8, wherein P is the n-octanol/water partition coefficient of said component; and (c) a malodour-preventing composition each of the components of which has a C $\log_{10}P$ of between I and 8, wherein P is the n-octanol/water partition coefficient of said component to be in admixture with said slurry by means of admixing said second functional composition (A) with said aqueous solution or (B) with said slurry;
vii. placing said microcapsule slurry into said enclosure;
viii. situating said enclosure whereby the nozzle exit ports of the at least one extemally-located spray nozzle are each substantially located in a plane substantially parallel to and opposite said one or more exposed surface areas of said one or more fabric articles at a substantially perpendicular mean distance of from about 0 to about 3 meters from said one or more exposed surface areas of said one or more fabric articles;
ix. applying sufficient pressure to said slurry located within said enclosure to enable said slurry to be sprayed through said one or more nozzle exit ports onto said one or more exposed surface areas of said one or more fabric articles whereby said microcapsules are effectively adhered to said one or more exposed surface areas of said one or more fabric articles thereby forming one or more microcapsule-fixed fabric article surface areas; and whereby (a) the concentration of the functional substance contained in the slurry is from about 0.03% to about 0.8% immediately prior to the step ix and (b) subsequent to the step ix when said microcapsule-fixed fabric article surface areas are rubbed, said microcapsules rupture, thereby emitting said first functional substance.

2. The process of claim 1 wherein a non-encapsulated second functional substance selected from the group consisting of (a) a fragrance composition each of the components of which has a C $\log_{10}P$ of between 1 and 8, wherein P is

the n-octanol/water partition coefficient of said component; (b) a malodour counteracting composition each of the components of which has a C $log_{10}P$ of between 1 and 8, wherein P is the n-octanol/water partition coefficient of said component; and (c) a malodour-preventing composition each of the components of which has a C $log_{10}P$ of between 1 and 8, wherein P is the n-octanol/water partition coefficient of said component is caused to be in admixture with said slurry by means of admixing said second functional composition (A) with said aqueous solution or (B) with said slurry.

**3.** The process of claim 1 wherein said first functional substance comprises a mixture of zinc ricinoleate or a solution thereof and a substituted monocyclic organic compound selected from the group consisting of:

1-cyclohexylethan-1-yl butyrate;
1-cyclohexylethan-1-yl acetate;
1-cyclohexylethan-1-ol;
1-(4'-methylethyl)cyelohexylethan-1-yl propionate; and
2'-hydroxy-1'-ethyl(2-phenoxy)acetate.

**4.** The process of claim 2 wherein said first functional substance and said second functional substance each comprises a mixture of zinc ricinoleate or a solution thereof and a substituted monocyclic organic compound selected from the group consisting of:

1-cyclohexylethan-1-yl butyrate;
1-cyclohexylethan-1-yl acetate;
1-cyclohexylethan-1-ol;
1-(4'-methylethyl)cyclohexylethan-1-yl propionate; and
2'-hydroxy-1'-ethyl(2-phenoxy)acetate.

**5.** The process of claim 1 wherein said first functional substance is a fragrance composition which effects malodour suppression.

**6.** The process of claim 2 wherein said first functional substance is a fragrance composition which effects malodour suppression and said second functional substance is a fragrance composition which effects malodour suppression.

**7.** The process of claim 2 wherein said first functional substance comprises a mixture of zinc ricinoleate or a solution thereof and a substituted monocyclic organic compound selected from the group consisting of:

1-cyclohexylethan-1-yl butyrate;
1-cyclohexylethan-1-yl acetate;
1-cyclohexylethan-1-ol;
1-(4'-methylethyl)cyclohexylethan-1-yl propionate; and
2'-hydroxy-1'-ethyl(2-phenoxy)acetate

and said second functional substance is a fragrance composition which effects malodour suppression.

**8.** The process of claim 2 wherein said first functional substance is a fragrance composition which effects malodour suppression and said second functional substance comprises a mixture of zinc ricinoleate or a solution thereof and a substituted monocyclic organic compound selected from the group consisting of:

1-cyclohexylethan-1-yl butyrate;
1-cyclohexylethan-1-yl acetate;
1-cyclohexylethan-1-ol ;
1-(4'-methylethyl)cyclohexylethan-1-yl propionate; and
2'-hydroxy-1'-ethyl(2-phenoxy)acetate.

**9.** The process of claim 1 wherein the scaled aesthetically-pleasing fragrance intensity, Y of the functional substance vs. time in days, X, subsequent to rubbing is defined according to the algorithm:

$$Y = \alpha X^3 + \beta X^2 + \gamma X + \delta$$

wherein
$-0.05 \leq \alpha \leq +0.03$;
$-2.0 \leq \beta \leq +0.3$;
$-1.0 \leq \gamma \leq +5.0$; and
$-1.0 \leq \delta \leq +5.0$.

**10.** The process of claim 1 wherein at least a finite portion of the microcapsules is in contact with a polymeric silicone phospholipid.

**11.** The process of claim 10 wherein the polymeric silicone phospholipid is prepared by the phosphation reaction of a terminal dimethicone copolyol with a phosphating agent followed by neutralization of the phosphate with base followed by a condensation reaction with an epihalohydrin followed by conducting a n-alkylation reaction with an amine.

**12.** A microcapsule consisting essentially of a melamine-formaldehyde polymeric shell and enclosed within the shell a composition comprising a mixture of zinc ricinoleate or a solution thereof and a substituted monocyclic organic compound selected from the group consisting of:

1-cyclohexylethan-1-yl butyrate;
1-cyclohexylethan-1-yl acetate;
1-cyclohexylethan-1-ol;
1-(4'-methylethyl)cyclohexylethan-1-yl propionate; and
2'-hydroxy-1'-ethyl(2-phenoxy)acetate.

**13.** A slurry comprising an aqueous base, a microcapsule suspended in said base consisting essentially of a melamine-formaldehyde polymeric shell; enclosed within the shell a functional ingredient selected from the group consisting of (a) a fragrance composition each of the components of which has a C $\log_{10}P$ of between 2.5 and 8, wherein P is the n-octanol/water partition coeffiicent of said component, and (b) a malodour counteracting composition each of the components of which has a C $\log_{10}P$ of between 2.5 and 8, wherein P is the n-octanol/water partition coeffiicent of said component, and in contact with said microcapsule, a polymeric silicone phospholipid.

**14.** The slurry of claim 13 wherein the polymeric silicone phospholipid is prepared by the phosphation reaction of a terminal dimethicone copolyol with a phosphating agent followed by neutralization of the phosphate with base followed by a condensation reaction with an epihalohydrin followed by conducting a n-alkylation reaction with an amine.

**15.** The process of claim 1 wherein the aqueous solution provided in step iv consists essentially of (a) from about 80 to about 93 parts by weight of water; (b) from about 4 to about 8 parts by weight of ethanol; (c) from about 2 to about 3 parts by weight of non-ionic surfactant; (d) from about 0.05 to about 0.5 parts by weight of preservative; (e) from about 0.1 to about 2 parts by weight of silicone polymer and (f) from about 0.05 to about 0.1 parts by weight of suspending agent.

**16.** The process of claim 15 wherein the non-ionic surfactant is a mixture of the hydroxy-octaethoxy ethers of n-nonanol and n-undecanol; the preservative is hexahydro-1,3,5-tris(2-hydroxyethyl)-s-triazine and the suspending agent is selected from the group consisting of attapulgite clay, xanthan gum, hydroxypropyl cellulose having a molecular weight of from about 50,000 to about 800,000, colloidal silica, ethyl cellulose having a particle size of from about 0.004 microns to about 0.130 microns, a surface area of from about 100 $m^2$ per gram to about 500 $m^2$ per gram and a density of from about 1.0 to about 4.0 pounds per $ft^3$.

**17.** The slurry of claim 13 wherein the aqueous base consists essentially of (a) from about 80 to about 93 parts by weight of water; (b) from about 4 to about 8 parts by weight of ethanol; (c) from about 2 to about 3 parts by weight of non-ionic surfactant; (d) from about 0.05 to about 0.5 parts by weight of preservative; (e) from about 0.1 to about 2 parts by weight of silicone polymer and (f) from about 0.05 to about 0.1 parts by weight of suspending agent.

**18.** The slurry of claim 17 wherein the non-ionic surfactant is a mixture of the hydroxy-octaethoxy ethers of n-nonanol and n-undecanol; the preservative is hexahydro-1,3,5-tris(2-hydroxyethyl)-s-triazine and the suspending agent is selected from the group consisting of attapulgite clay, xanthan gum, hydroxypropyl cellulose having a molecular weight of from about 50,000 to about 800,000, colloidal silica, ethyl cellulose having a particle size of from about 0.004 microns to about 0.130 microns, a surface area of from about 100 $m^2$ per gram to about 500 $m^2$ per gram and a density of from about 1.0 to about 4.0 pounds per $ft^3$.

**19.** The slurry of claim 13 wherein admixed with said base is a non-confined fragrance composition each of the components of which has a C $log_{10}P$ of between 1 and 8, wherein P is the n-octanol/water partition coeffiicent of said component, and in contact with said non-confined fragrance composition, a polymeric silicone phospholipid.

**20.** The slurry of claim 19 wherein the aqueous base consists essentially of (a) from about 80 to about 93 parts by weight of water; (b) from about 4 to about 8 parts by weight of ethanol; (c) from about 2 to about 3 parts by weight of non-ionic surfactant; (d) from about 0.05 to about 0.5 parts by weight of preservative; (e) from about 0.1 to about 2 parts by weight of silicone polymer and (f) from about 0.05 to about 0.1 parts by weight of suspending agent.

**21.** The slurry of claim 20 wherein the non-ionic surfactant is a mixture of the hydroxy-octaethoxy ethers of n-nonanol and n-undecanol; the preservative is hexahydro-1,3,5-tris(2-hydroxyethyl)-s-triazine and the suspending agent is selected from the group consisting of attapulgite clay, xanthan gum, hydroxypropyl cellulose having a molecular weight of from about 50,000 to about 800,000, colloidal silica, ethyl cellulose having a particle size of from about 0.004 microns to about 0.130 microns, a surface area of from about 100 $m^2$ per gram to about 500 $m^2$ per gram and a density of from about 1.0 to about 4.0 pounds per $ft^3$.

## Patentansprüche

**1.** Verfahren zum im Wesentlichen (a) Verleihen von Duft mindestens einem Gegenstand aus Gewebe für einen verlängerten Zeitraum und/oder (b) im Wesentlichen Beseitigen von schlechten Gerüchen von einem Gegenstand aus Gewebe für einen verlängerten Zeitraum und/oder (c) Überdecken von schlechten Gerüchen, die von mindestens einem Gegenstand aus Gewebe abgegeben werden, für einen verlängerten Zeitraum und/oder (d) Verhindern des Bildens von schlechtem Geruch in mindestens einem Gegenstand aus Gewebe für einen verlängerten Zeitraum, umfassend die Schritte:

i. des Bereitstellens einer oder mehrerer exponierter Oberflächen eines oder mehrerer Gegenstände aus Gewebe;
ii. des Bereitstellens einer Umfassung, ausgerüstet mit mindestens einem druckaktivierten Luftzerstäuber mit einer außen angeordneten Sprühdüse, die mit dem Inneren der Umfassung in Verbindung steht, wobei die Düse 1 bis zu einer Mehrzahl von Düsenausgangsöffnungen mit effektiven Durchmessern $D_{NPi}$ der Düsenausgangsöffnungen aufweist;
iii. des Herstellens einer Mehrzahl von Mikrokapseln, wobei jede dieser aus einer zerreißbaren äußeren Wand aus einem Melamin-Formlaldehydpolymer zusammengesetzt ist, das etwa 10 Gew.-% bis etwa 30 Gew.-% einer ersten Funktionssubstanz, ausgewählt aus der Gruppe, bestehend aus (a) einer Duftzusammensetzung, wobei jede der Komponenten dieser einen C $log_{10}P$ zwischen 2,5 und 8 aufweist, wobei P der n-Octanol/Wasser-Verteilungskoeffizient der Komponente ist, (b) eine schlechtem Geruch entgegenwirkende Zusammensetzung, wobei jede der Komponenten dieser einen C $log_{10}P$ zwischen 2,5 und 8 aufweist, wobei P der n-Octanol/Wasser-Verteilungskoeffizient der Komponente ist, und (c) eine schlechten Geruch verhindernde Zusammensetzung, wobei jede der Komponenten dieser einen C $log_{10}P$ zwischen 2,5 und 8 aufweist, wobei P der n-Octanol/Wasser Verteilungskoeffizient der Komponente ist, einschließt, wobei jede der Mikrokapseln einen durchschnittlichen effektiven Durchmesser $\overline{D_{MC}}$ von etwa 5 Mikrometer bis etwa 80 Mikrometer aufweist, wobei jede dieser Mikrokapseln einen wirksamen Durchmesser $D_{MCi}$ aufweist, wobei der kleinste der $D_{NPi}$ wesentlich größer als der größte der $D_{MCi}$ ist, wobei $\overline{D_{MC}} = \frac{1}{N}\sum D_{MCi}$ und N die Zahl der Mikrokapseln in der Aufschlämmung darstellt, die in der Umfassung enthalten ist;
iv. des Bereitstellens einer wäßrigen Lösung, umfassend (a) Wasser, (b) ein kompatibles Lösungsmittel, ausgewählt aus der Gruppe, bestehend aus Ethanol, dem Mono-$C_1$ oder -$C_2$-Ether eines Mono-, Di-, oder Tri-1,2-Propylenglykols und dem Di-$C_1$-oder -$C_2$-Ether eines Mono-, Di- oder Tri-1,2-propylenglykols, (c) ein Silikonpolymer, (d) ein kompatibles nicht-ionisches grenzflächenaktives Mittel, (e) ein kompatibles Konservierungsmittel und (f) ein kompatibles Suspensionsmittel;

v. des Mischens der Mehrzahl von Mikrokapseln mit der wäßrigen Lösung mit einem Anteil von etwa 0,1 Gew.-% bis etwa 0,4 Gew.-% Mikrokapseln, basierend auf dem Gewicht der wäßrigen Lösung, wodurch eine Mikrokapselaufschlämmung gebildet wird, wobei die Mikrokapseln in der Aufschlämmung suspendiert sind und jede der Mikrokapseln eine Absetzgeschwindigkeit $V_S$ in der Aufschlämmung von gleich oder etwa 0 aufweist;

vi. gegebenenfalls des Verursachens, dass eine nicht-eingeschlossene zweite Funktionssubstanz, ausgewählt aus der Gruppe, bestehend aus (a) einer Duftzusammensetzung, wobei jede der Komponenten dieser einen C $\log_{10}$P zwischen 1 und 8 aufweist, wobei P der n-Octanol/Wasser-Verteilungskoeffizient der Komponente ist, (b) eine schlechtem Geruch entgegenwirkende Zusammensetzung, wobei jede der Komponenten dieser einen C $\log_{10}$P zwischen 1 und 8 aufweist, wobei P der n-Octanol/Wasser-Verteilungskoeffizient der Komponente ist, und (c) eine schlechten Geruch verhindernde Zusammensetzung, wobei jede der Komponenten dieser einen C $\log_{10}$P zwischen 1 und 8 aufweist, wobei P der n-Octanol/Wasser-Verteilungskoeffizient der Komponente ist, im Gemisch mit der Aufschlämmung mittels des Mischens der zweiten Funktionszusammensetzung (A) mit der wäßrigen Lösung oder (B) mit der Aufschlämmung vorliegt;

vii. des Einbringens der Mikrokapselaufschlämmung in die Umfassung;

viii. des Aufstellens der Umfassung, wodurch die Düsenausgängsöffnungen der mindestens einen außen angeordneten Sprühdüse jeweils im Wesentlichen in einer Ebene im Wesentlichen parallel zu und gegenüberliegend der einen oder mehreren exponierten Oberflächen des einen oder der mehreren Gegenstände aus Gewebe bei einem im Wesentlichen senkrechten mittleren Abstand von etwa 0 bis 3 Metern von der einen oder den mehreren exponierten Oberflächen des einen oder der mehreren Gegenstände aus Gewebe angeordnet sind;

ix. des Anlegens von ausreichendem Druck auf die innerhalb der Umfassung angeordnete Aufschlämmung, um zu ermöglichen, dass die Aufschlämmung durch die eine oder mehreren Düsenausgangsöffnungen auf die eine oder mehreren exponierten Oberflächen des einen oder der mehreren Gegenstände aus Gewebe gesprüht wird, wodurch die Mikrokapseln wirksam an die eine oder mehreren exponierten Oberflächen des einen oder der mehreren Gegenstände aus Gewebe angehaftet werden, wodurch eine oder mehrere Oberflächen des Gegenstands aus Gewebe mit fixierten Mikrokapseln gebildet werden; und

wobei (a) die Konzentration der in der Aufschlämmung enthaltenen Funktionssubstanz direkt vor dem Schritt ix von etwa 0,03% bis etwa 0,8% beträgt, und (b) nach dem Schritt ix, wenn die Oberflächen des Gegenstands aus Gewebe, auf denen Mikrokapseln fixiert sind, gerieben werden, die Mikrokapseln reißen, wodurch die erste Funktionssubstanz abgegeben wird.

**2.** Verfahren nach Anspruch 1, wobei verursacht wird, dass eine nicht-eingeschlossene zweite Funktionssubstanz, ausgewählt aus der Gruppe, bestehend aus (a) einer Duftzusammensetzung, wobei jede der Komponenten dieser einen C $\log_{10}$P zwischen 1 und 8 aufweist, wobei P der n-Octanol/Wasser-Verteilungskoeffizient der Komponente ist, (b) eine schlechtem Geruch entgegenwirkende Zusammensetzung, wobei jede der Komponenten dieser einen C $\log_{10}$P zwischen 1 und 8 aufweist, wobei P der n-Octanol/Wasser-Verteilungskoeffizient der Komponente ist, und (c) eine schlechten Geruch verhindernde Zusammensetzung, wobei jede der Komponenten dieser einen C $\log_{10}$P zwischen 1 und 8 aufweist, wobei P der n-Octanol/Wasser-Verteilungskoeffizient der Komponente ist, im Gemisch mit der Aufschlämmung mittels des Mischens der zweiten Funktionszusammensetzung (A) mit der wäßrigen Lösung oder (B) mit der Aufschlämmung vorliegt.

**3.** Verfahren nach Anspruch 1, wobei die erste Funktionssubstanz ein Gemisch aus Zinkricinoleat oder einer Lösung davon und einer substituierten monocyclischen organischen Verbindung, ausgewählt aus der Gruppe, bestehend aus
1-Cyclohexylethan-1-yl butyrat,
1-Cyclohexylethan-1-yl acetat,
1-Cyclohexyfethan-1-ol,
1-(4'-Methylethyl)cyclohexylethan-1-yl propionat und
2'-Hydroxy-1'-ethyl(2-phenoxy)acetat
umfasst.

**4.** Verfahren nach Anspruch 2, wobei die erste Funktionssubstanz und die zweite Funktionssubstanz jeweils ein Gemisch aus Zinkricinoleat oder einer Lösung davon und einer substituierten monocyclischen organischen Verbindung, ausgewählt aus der Gruppe, bestehend aus
1-Cyclohexylethan-1-yl butyrat,

1-Cyclohexylethan-1-yl acetat,
1-Cyclohexylethan-1-ol,
1-(4'-Methylethyl)cyclohexylethan-1-yl propionat und
2' -Hydroxy-1'-ethyl(2 -phenoxy)acetat
umfassen.

5. Verfahren nach Anspruch 1, wobei die erste Funktionssubstanz eine Duftzusammensetzung ist, welche die Unterdrückung von schlechtem Geruch bewirkt.

6. Verfahren nach Anspruch 2, wobei die erste Funktionssubstanz eine Duftzusammensetzung ist, welche die Unterdrückung von schlechtem Geruch bewirkt, und die zweite Funktionssubstanz eine Duftzusammensetzung ist, welche die Unterdrückung von schlechtem Geruch bewirkt.

7. Verfahren nach Anspruch 2, wobei die erste Funktionssubstanz ein Gemisch aus Zinkricinoleat oder einer Lösung davon und einer substituierten monocyclischen organischen Verbindung, ausgewählt aus der Gruppe, bestehend aus
1-Cyclohexylethan-1-yl butyrat,
1-Cyclohexylethan-1-yl acetat,
1-Cyclohexylethan-1-ol,
1-(4'-Methylethyl)cyclohexylethan-1-yl propionat und
2'-Hydroxy-1'-ethyl(2-phenoxy)acetat
umfasst, und die zweite Funktionssubstanz eine Duftzusammensetzung ist, welche die Unterdrückung von schlechtem Geruch bewirkt.

8. Verfahren nach Anspruch 2, wobei die erste Funktionssubstanz eine Duftzusammensetzung ist, welche die Unterdrückung von schlechtem Geruch bewirkt, und die zweite Funktionssubstanz ein Gemisch aus Zinkricinoleat oder einer Lösung davon und einer substituierten monocyclischen organischen Verbindung, ausgewählt aus der Gruppe, bestehend aus
1-Cyclohexylethan-1-yl butyrat,
1-Cyclohexylethan-1-yl acetat,
1-Cyclohexylethan-1-ol,
1-(4'-Methylethyl)cyclohexylethan-1-yl propionat und
2'-Hydroxy-1'-ethyl(2-phenoxy)acetat
umfasst.

9. Verfahren nach Anspruch 1, wobei die skalierfe ästhetisch-angenehme Duftintensität Y der Funktionssubstanz gegen die Zeit in Tagen X nach dem Reiben gemäß dem folgenden Algorithmus definiert ist:

$$Y=\alpha X^3+\beta X^2+\gamma X+\delta$$

wobei
$-0{,}05\leq\alpha\leq+0{,}03$;
$-2{,}0\leq\beta\leq+0{,}3$;
$-1{,}0\leq\gamma\leq+5{,}0$; und
$-1{,}0\leq\delta\leq+5{,}0$.

10. Verfahren nach Anspruch 1, wobei mindestens ein endlicher Teil der Mikrokapseln mit einem polymeren Silikonphospholipid in Kontakt steht.

11. Verfahren nach Anspruch 10, wobei das polymere Silikonphospholipid durch die Phosphatierungsreaktion eines endständigen Dimethiconcopolyols mit einem Phosphatiermittel, gefolgt von der Neutralisation des Phosphats mit Base, gefolgt von einer Kondensationsreaktion mit einem Epihalohydrin, gefolgt von der Durchführung einer n-Alkylierungsreaktion mit einem Amin, hergestellt wird.

12. Mikrokapsel, im Wesentlichen bestehend aus einer polymeren MelaminFormaldehyd-Hülle, und wobei innerhalb der Hülle eine Zusammensetzung eingeschlossen ist, umfassend ein Gemisch aus Zinkricinoleat oder einer Lösung

davon und einer substituierten monocyclischen organischen Verbindung, ausgewählt aus der Gruppe, bestehend aus
1-Cyclohexylethan-1-yl butyrat,
1-Cyclohexylethan-1-yl acetat,
1-Cyclohexylethan-1-ol,
1-(4'-Methylethyl)cyclohexylethan-1-yl propionat und
2'-Hydroxy-1'-ethyl(2-phenoxy)acetat.

**13.** Aufschlämmung, umfassend eine wäßrige Base, eine in der Base suspendierte Mikrokapsel, im Wesentlichen bestehend aus einer polymeren Melamin-Formaldehyd-Hülle, wobei innerhalb der Hülle ein Funktionsbestandteil, ausgewählt aus der Gruppe, bestehend aus (a) einer Duftzusammensetzung, wobei jede der Komponenten dieser einen C $\log_{10}P$ zwischen 2,5 und 8 aufweist, wobei P der n-Oetanol/Wasser-Vorteilungskoeffizient der Komponente ist, und (b) eine schlechtem Geruch entgegenwirkende Zusammensetzung, wobei jede der Komponenten dieser einen C $\log_{10}P$ zwischen 2,5 und 8 aufweist, wobei P der n-Octanol/Wasser-Verteilungskoeffizient der Komponente ist, eingeschlossen ist, und wobei ein polymeres Silikonphospholipid mit der Mikrokapsel in Kontakt steht.

**14.** Aufschlämmung nach Anspruch 13, wobei das polymere Silikonphospholipid durch die Phosphatierungsreaktion eines endständigen Dimethiconcopolyols mit einem Phosphatiermittel, gefolgt von der Neutralisation des Phosphats mit Base, gefolgt von einer Kondensationsreaktion mit einem Epihalohydrin, gefolgt von der Durchführung einer n-Alkylierungsreaktion mit einem Amin, hergestellt ist.

**15.** Verfahren nach Anspruch 1, wobei die in Schritt iv bereitgestellte wäßrige Lösung im Wesentlichen aus (a) etwa 80 bis etwa 93 Gewichtsteilen Wasser, (b) etwa 4 bis etwa 8 Gewichtsteilen Ethanol, (c) etwa 2 bis etwa 3 Gewichtsteilen nicht-ionisches grenzflächenaktives Mittel, (d) etwa 0,05 bis etwa 0,5 Gewichtsteilen Konservierungsmittel, (e) etwa 0,1 bis etwa 2 Gewichtsteilen Silikonpolymer und (f) etwa 0,05 bis etwa 0,1 Gewichtsteilen Suspensionsmittel besteht.

**16.** Verfahren nach Anspruch 15, wobei das nicht-ionische grenzflächenaktive Mittel ein Gemisch der Hydroxy-octaethoxyether von n-Nonanol und n-Undecanol ist, das Konservierungsmittel Hexahydro-1,3;5-tris(2-hydroxyethyl)-s-triazin ist und das Suspensionsmittel aus der Gruppe, bestehend aus Attapulgitton, Xanthangummi, Hydroxypropylcellulose mit einem Molekulargewicht von etwa 50.000 bis etwa 800.000, kolloidalem Siliziumdioxid, Ethylcellulose mit einer Teilchengröße von etwa 0,004 Mikrometer bis etwa 0,130 Mikrometer, einer Oberfläche von etwa 100 $m^2$ pro Gramm bis etwa 500 $m^2$ pro Gramm und einer Dichte von etwa 1,0 bis etwa 4,0 Pfund pro $ft^3$, ausgewählt ist.

**17.** Aufschlämmung nach Anspruch 13, wobei die wäßrige Base im Wesentlichen aus (a) etwa 80 bis etwa 93 Gewichtsteilen Wasser, (b) etwa 4 bis etwa 8 Gewichtsteilen Ethanol, (c) etwa 2 bis etwa 3 Gewichtsteilen nicht-ionisches grenzflächenaktives Mittel, (d) etwa 0,05 bis etwa 0,5 Gewichtsteilen Konservierungsmittel, (e) etwa 0,1 bis etwa 2 Gewichtsteilen Silikonpolymer und (f) etwa 0,05 bis etwa 0,1 Gewichtsteilen Suspensionsmittel besteht.

**18.** Aufschlämmung nach Anspruch 17, wobei das nicht-ionische grenzflächenaktive Mittel ein Gemisch der Hydroxy-octaethoxyether von n-Nonanol und n-Undecanol ist, das Konservierungsmittel Hexahydro-1,3,5-tris(2-hydroxyethyl)-s-triazin ist und das Suspensionsmittel aus der Gruppe, bestehend aus Attapulgitton, Xanthangummi, Hydroxypropylcellulose mit einem Molekulargewicht von etwa 50.000 bis etwa 800.000, kolloidalem Siliziumdioxid, Ethylcellulose mit einer Teilchengröße von etwa 0,004 Mikrometer bis etwa 0,130 Mikrometer, einer Oberfläche von etwa 100 $m^2$ pro Gramm bis etwa 500 $m^2$ pro Gramm und einer Dichte von etwa 1,0 bis etwa 4,0 Pfund pro $ft^3$, ausgewählt ist.

**19.** Aufschlämmung nach Anspruch 13, wobei mit der Base eine nicht-eingeschlossene Duftzusammensetzung gemischt ist, wobei jede Komponente dieser einen C $\log_{10}P$ zwischen 1 und 8 aufweist, wobei P der n-Octanol/Wasser-Verteilungskoeffizient der Komponente ist, und wobei ein polymeres Silikonphospholipid mit der nicht-eingeschlossenen Duftzusammensetzung in Kontakt steht.

**20.** Aufschlämmung nach Anspruch 19, wobei die wäßrige Base im Wesentlichen aus (a) etwa 80 bis etwa 93 Gewichtsteilen Wasser, (b) etwa 4 bis etwa 8 Gewichtsteilen Ethanol, (c) etwa 2 bis etwa 3, Gewichtsteilen nicht-ionisches grenzflächenaktives Mittel, (d) etwa 0,05 bis etwa 0,5 Gewichtsteilen Konservierungsmittel, (e) etwa 0,1 bis etwa 2 Gewichtsteilen Silikonpolymer und (f) etwa 0,05 bis etwa 0,1 Gewichtsteilen Suspensionsmittel besteht.

**21.** Aufschlämmung nach Anspruch 20, wobei das nicht-ionische grenzflächenaktive Mittel ein Gemisch der Hydroxy-octaethoxyether von n-Nonanol und n-Undecanol ist, das Konservierungsmittel Hexahydro-1,3,5-tris(2-hydroxyethyl)-s-triazin ist und das Suspensionsmittel aus der Gruppe, bestehend aus Attapulgitton, Xanthangummi, Hydroxypropylcellulose mit einem Molekulargewicht von etwa 50.000 bis etwa 800.000, kolloidalem Siliziumdioxid, Ethylcellulose mit einer Teilchengröße von etwa 0,004 Mikrometer bis etwa 0,130 Mikrometer, einer Oberfläche von etwa 100 $m^2$ pro Gramm bis etwa 500 $m^2$ pro Gramm und einer Dichte von etwa 1,0 bis etwa 4,0 Pfund pro $ft^3$, ausgewählt ist.

## Revendications

**1.** Procédé pour (a) sensiblement communiquer un parfum à au moins un article textile et/ou (b) sensiblement y éliminer les mauvaises odeurs et/ou (c) couvrir les mauvaises odeurs qui s'en dégagent et/ou (d) y empêcher la formation de mauvaises odeurs, sur une période de temps prolongée, comprenant les étapes suivantes :

i. on met en oeuvre une ou plusieurs zones de surface exposées d'un article textile ou plus;
ii. on met en oeuvre une enceinte équipée d'au moins un pulvérisateur d'air activé par pression ayant une buse de pulvérisation située à l'extérieur communiquant avec l'intérieur de ladite enceinte, ladite buse ayant 1 à une pluralité d'orifices de sortie de la buse présentant des diamètres effectifs d'orifices de sortie de buse $D_{NPi}$;
iii. on prépare une pluralité de microcapsules dont chacune est composée d'une paroi externe frangible d'un polymère de mélamine-formaldéhyde renfermant environ 10% en poids à environ 30% en poids d'une première substance fonctionnelle choisie dans le groupe constitué de (a) une composition de parfum dont chacun des composants a un $Clog_{10}P$ entre 2, 5 et 8, P étant le coefficient de partage n-octanol/eau dudit composant; (b) une composition neutralisant les mauvaises odeurs dont chacun des composants a un $Clog_{10}P$ entre 2,5 et 8, P étant le coefficient de partage n-octanol/eau dudit composant; et (c) un composant empêchant les mauvaises odeurs dont chacun des composants a un $Clog_{10}P$ entre 2,5 et 8, P étant le coefficient de partage n-octanol/eau, chacune desdites microcapsules ayant un diamètre effectif moyen $\overline{D_{MC}}$ d'environ 5 micromètres à environ 80 micromètres, chaque microcapsule ayant un diamètre effectif de $D_{MCi}$, dans lequel le plus petit des $D_{NPi}$ est sensiblement supérieur au plus grand des $D_{MCi}$, dans lequel $\overline{D_{MC}} = \frac{1}{N}\sum D_{MCi}$ et N représente le nombre de microcapsules dans la suspension contenue dans ladite enceinte;
iv. on met en oeuvre une solution aqueuse comprenant (a) de l'eau, (b) un solvant compatible choisi dans le groupe constitué de l'éthanol, du monoéther en $C_1$ ou $C_2$ d'un mono-, di-, ou tri-1,2-propylèneglycol et du diéther en $C_1$ ou $C_2$ d'un mono-, di- ou tri-1,2-propylèneglycol, (c) un polymère de silicone, (d) un agent tensioactif non ionique compatible, (e) un conservateur compatible et (f) un agent de mise en suspension compatible;
v. on mélange ladite pluralité de microcapsules à ladite solution aqueuse à un niveau d'environ 0,1% en poids à environ 0,4% en poids de microcapsules par rapport au poids de la solution aqueuse, formant de la sorte une suspension de microcapsules dans laquelle lesdites microcapsules sont suspendues dans ladite suspension et chacune desdites microcapsules a une vitesse de sédimentation dans ladite suspension, $V_S$, égale à environ 0;
vi. on soumet éventuellement une seconde substance fonctionnelle non encapsulée choisie dans le groupe constitué de (a) une composition de parfum dont chacun des composants a un C $log_{10}P$ entre 1 et 8, P étant le coefficient de partage n-octanol/eau dudit composant; (b) une composition neutralisant les mauvaises odeurs dont chacun des composants a un C $log_{10}P$ entre 1 et 8, P étant le coefficient de partage n-octanol/eau dudit composant; et (c) une composition empêchant les mauvaises odeurs dont chacun des composants a chacun un C $log_{10}P$ entre 1 et 8, P étant le coefficient de partage n-octanol/eau dudit composant, en mélange à ladite suspension en mélangeant ladite seconde composition fonctionnelle (A) à ladite solution aqueuse ou (B) à ladite suspension;
vii. on place ladite suspension de microcapsules dans ladite enceinte;
viii. on positionne ladite enceinte de sorte que les orifices de sortie d'au moins une des tuyères de pulvérisation située à l'extérieur soient chacun sensiblement situés dans un plan sensiblement parallèle et opposé à ladite une ou plusieurs zones de surface exposées dudit un ou plusieurs articles textiles à une distance moyenne sensiblement perpendiculaire d'environ 0 à environ 3 mètres de ladite une ou plusieurs zones de surface exposées dudit un ou plusieurs articles textiles;
ix. on applique une pression suffisante à ladite suspension située à l'intérieur de ladite enceinte pour permettre à ladite suspension d'être pulvérisée à travers un ou plusieurs orifices de sortie de buse sur lesdites une ou plusieurs zones de surface exposées de sorte que lesdites microcapsules soient efficacement collées à ladite

une ou plusieurs surfaces exposées dudit un ou plusieurs articles textiles formant de la sorte une ou plusieurs zones de surface d'articles textile à microcapsules fixées; et

où (a) la concentration de la substance fonctionnelle contenue dans la suspension soit d'environ 0,03% à environ 0,8% immédiatement avant l'étape ix et (b) à la suite de l'étape ix, lorsque lesdites zones de surface d'articles textiles à microcapsules fixées sont frottées, la rupture desdites microcapsules émettant de la sorte ladite première substance fonctionnelle.

**2.** Procédé selon la revendication 1, dans lequel une seconde substance fonctionnelle non encapsulée choisie dans le groupe constitué de (a) une composition de parfum dont chaque composant a un C $\log_{10}P$ entre 1 et 8, P étant le coefficient de partage n-octanol/eau dudit composant; (b) une composition neutralisant les mauvaises odeurs dont chacun des composants a un C $\log_{10}P$ entre 1 et 8, P étant le coefficient de partage n-octanol/eau dudit composant; et (c) une composition empêchant les mauvaises odeurs, dont chacun des composants a un C $\log_{10}P$ entre 1 et 8, P étant le coefficient de partage n-octanol/eau dudit composant, est soumise à un mélange à ladite suspension en mélangeant ladite seconde composition fonctionnelle (A) à ladite solution aqueuse ou (B) à ladite suspension.

**3.** Procédé selon la revendication 1, dans lequel ladite première substance fonctionnelle comprend un mélange de ricinoléate de zinc ou une solution de celui-ci et d'un composé organique monocyclique substitué choisi dans le groupe constitué par :

butyrate de 1-cyclohexyléthan-1-yle;
acétate de 1-cyclohexyléthan-1-yle;
1-cyclohexyléthan-1-ol;
propionate de 1-(4'-méthyléthyl)cyclohexyléthan-1-yle; et
(2-phénoxy)acétate de 2'-hydroxy-1'-éthyle.

**4.** Procédé selon la revendication 2, dans lequel ladite première substance fonctionnelle et ladite seconde substance fonctionnelle comprennent chacune un mélange de ricinoléate de zinc ou une solution de celui-ci et d'un composé organique monocyclique substitué choisi dans le groupe constitué par :

butyrate de 1-cyclohexyléthan-1-yle;
acétate de 1-cyclohexyléthan-1-yle;
1-cyclohexyléthan-1-ol;
propionate de 1-(4'-méthyléthyl)cyclohexyléthan-1-yle ; et
(2-phénoxy)acétate de 2'-hydroxy-l'-éthyle.

**5.** Procédé selon la revendication 1, dans lequel ladite première substance fonctionnelle est une composition de parfum qui supprime les mauvaises odeurs.

**6.** Procédé selon la revendication 2, dans lequel ladite première substance fonctionnelle est une composition de parfum qui supprime les mauvaises odeurs et ladite seconde substance fonctionnelle est une composition de parfum qui supprime les mauvaises odeurs.

**7.** Procédé selon la revendication 2, dans lequel ladite première substance fonctionnelle comprend un mélange de ricinoléate de zinc ou une solution de celui-ci et d'un composé organique monocyclique substitué choisi dans le groupe constitué par:

butyrate de 1-cyclohexyléthan-1-yle;
acétate de 1-cyclohexyléthan-1-yle;
1-cyclohexyléthan-1-ol;
propionate de 1-(4'-méthyléthyl)cyclohexyléthan-1-yle; et
(2-phénoxy)acétate de 2'-hydroxy-1'-éthyle,
et ladite seconde substance fonctionnelle est une composition de parfum qui supprime les mauvaises odeurs.

**8.** Procédé selon la revendication 2, dans lequel ladite première substance fonctionnelle est une composition de parfum qui supprime les mauvaises odeurs et ladite seconde substance fonctionnelle comprend un mélange de ricinoléate de zinc ou une solution de celui-ci et d'un composé organique monocyclique substitué choisi dans le groupe constitué

par :

butyrate de 1-cyclohexyléthan-1-yle;
acétate de 1-cyclohexyléthan-1-yle;
1-cyclohexyléthan-1-ol;
propionate de 1-(4'-méthyléthyl)cyclohexyléthan-1-yle; et
(2-phénoxy)acétate de 2'-hydroxy-l'-éthyle.

**9.** Procédé selon la revendication 1, dans lequel l'intensité du parfum esthétiquement agréable à l'échelle, Y, de la substance fonctionnelle en fonction du temps en jours, X, après frottement est définie selon l'algorithme :

$$Y = \alpha X^3 + \beta X^2 + \gamma X + \delta$$

dans lequel :
$-0.05 \leq \alpha \leq +0.03$;
$-2.0 \leq \beta \leq +0.3$;
$-1.0 \leq \gamma \leq +5.0$ ; et
$-1.0 \leq \delta \leq +5.0$.

**10.** Procédé selon la revendication 1, dans lequel au moins une portion finie des microcapsules est en contact avec un phospholipide de silicone polymère.

**11.** Procédé selon la revendication 10, dans lequel le phospholipide de silicone polymère est préparé par réaction de phosphatation d'un copolyol de diméthicone terminal avec un agent de phosphatation suivie de la neutralisation du phosphate avec une base, suivie d'une réaction de condensation avec une épihalohydrine, suivie elle-même de la conduite d'une réaction de n-alkylation avec une amine.

**12.** Microcapsule constituée sensiblement d'une coque polymère de mélamine-formaldéhyde et d'une composition, enserrée dans la coque, comprenant un mélange de ricinoléate de zinc ou une solution de celui-ci et d'un composé organique monocyclique substitué choisi dans le groupe constitué des suivants :

butyrate de 1-cyclohexyléthan-1-yle;
acétate de 1-cyclohexyléthan-1-yle;
1-cyclohexyléthan-1-ol;
propionate de 1-(4'-méthyléthyl)cyclohexyléthan-1-yle; et
(2-phénoxya)cétate de 2'-hydroxy-1'-éthyle.

**13.** Suspension comprenant une base aqueuse, une microcapsule mise en suspension dans ladite base constituée essentiellement d'une coque polymère de mélamine-formaldéhyde; un ingrédient fonctionnel enserré dans la coque et choisi dans le groupe constitué de (a) une composition de parfum dont chaque composant a un $Clog_{10}P$ entre 2,5 et 8, P étant le coefficient de partage n-octanol/eau dudit composant, et (b) une composition neutralisant les mauvaises odeurs, dont chaque composant a un $Clog_{10}P$ entre 2,5 et 8, P étant le coefficient de partage n-octanol/eau dudit composant et, en contact avec ladite microcapsule, un phospholipide de silicone polymère.

**14.** Suspension selon la revendication 13, dans laquelle le phospholipide de silicone polymère est préparé par réaction de phosphatation d'un copolyol de diméthicone terminal avec un agent de phosphatation suivie de la neutralisation du phosphate avec une base, suivie elle-même d'une réaction de condensation avec une épihalohydrine, suivie de la conduite d'une réaction de n-alkylation avec une amine.

**15.** Procédé selon la revendication 1, dans lequel la solution aqueuse fournie à l'étape iv est essentiellement constituée de (a) environ 80 à environ 93 parties en poids d'eau; de (b) environ 4 à environ 8 parties en poids d'éthanol; de (c) environ 2 à environ 3 parties en poids d'un agent tensioactif non ionique; de (d) environ 0,05 à environ 0,5 partie en poids de conservateur; de (e) environ 0,1 à environ 2 parties en poids de polymère de silicone et de (f) environ 0,05 à environ 0,1 partie en poids d'agent de mise en suspension.

**16.** Procédé selon la revendication 15, dans lequel l'agent tensioactif non ionique est un mélange d'hydroxy-octaéthoxyéthers de n-nonanol et de n-undécanol; le conservateur est la hexahydro-1,3,5-tris(2-hydroxyéthyl)-s-triazine et l'agent de mise en suspension est choisi dans le groupe constitué de l'argile attapulgite, de la gomme xanthane, de l'hydroxypropylcellulose ayant un poids moléculaire d'environ 50 000 à environ 800 000, de la silice colloïdale, de l'éthylcellulose ayant granulométrie d'environ 0,004 μm à environ 0,130 μm, une surface spécifique d'environ 100 $m^2$ par gramme à environ 500 $m^2$ par gramme et une densité d'environ 16,018 à environ 64,074 kg par $m^3$ ( environ 1,0 à environ 4,0 livres par pied cube).

**17.** Suspension selon la revendication 13, dans laquelle la base aqueuse est essentiellement constituée de (a) environ 80 à environ 93 parties en poids d'eau; de (b) environ 4 à environ 8 parties en poids d'éthanol; de (c) environ 2 à environ 3 parties en poids d'agent tensioactif non ionique; de (d) environ 0,05 à environ 0,5 partie en poids de conservateur; de (e) environ 0,1 à environ 2 parties en poids de polymère de silicone et de (f) environ 0,05 à environ 0,1 partie en poids d'agent de mise en suspension.

**18.** Suspension selon la revendication 17, dans laquelle l'agent tensioactif non ionique est un mélange d'hydroxy-octaéthoxyéthers de n-nonanol et de n-undécanol; le conservateur est l'hexahydro-1,3,5-tris(2-hydroxyéthyl)-s-triazine et l'agent de mise en suspension est choisi dans le groupe constitué de l'argile attapulgite, de la gomme xanthane, de l'hydroxypropylcellulose ayant un poids moléculaire d'environ 50 000 à environ 800 000, de la silice colloïdale, de l'éthylcellulose ayant une granulométrie d'environ 0,004 μm à environ 0,130 μm, une surface spécifique d'environ 100 $m^2$ par g à environ 500 $m^2$ par g et une masse volumique d'environ 16,018 à environ 64,074 kg par $m^3$ (1,0 à 4,0 livres par pied cube).

**19.** Suspension selon la revendication 13, dans laquelle est mélangée à ladite base, une composition de parfum non confinée dont chaque composant a un $Clog_{10}P$ entre 1 et 8, P étant le coefficient de partage n-octanol/eau dudit composant et, en contact avec ladite composition de parfum non confinée, un phospholipide de silicone polymère.

**20.** Suspension selon la revendication 19, dans laquelle la base aqueuse est essentiellement constituée de (a) environ 80 à environ 93 parties en poids d'eau; de (b) environ 4 à environ 8 parties en poids d'éthanol; de (c) environ 2 à environ 3 parties en poids d'agent tensioactif non ionique; de (d) environ 0,05 à environ 0,5 partie en poids de conservateur; de (e) environ 0,1 à environ 2 parties en poids de polymère de silicone et de (f) environ 0,05 à environ 0,1 partie en poids d'agent de mise en suspension.

**21.** Suspension selon la revendication 20, dans laquelle l'agent tensioactif non ionique est un mélange d'hydroxy-octaéthoxyéthers de n-nonanol et de n-undécanol; le conservateur est l'hexahydro-1,3,5-tris(2-hydroxyéthyl)-s-triazine et l'agent de mise en suspension est choisi dans le groupe constitué de l'argile attapulgite, de la gomme xanthane, de l'hydroxypropylcellulose ayant un poids moléculaire d'environ 50 000 à environ 800 000, de la silice colloïdale, de l'éthylcellulose ayant une granulométrie d'environ 0,004 μm à environ 0,130 μm, une surface spécifique d'environ 100 $m^2$ par g à environ 500 $m^2$ par g et une masse volumique d'environ 16,018 à environ 64,074 kg par $m^3$ (1,0 à 4,0 livres par pied cube).

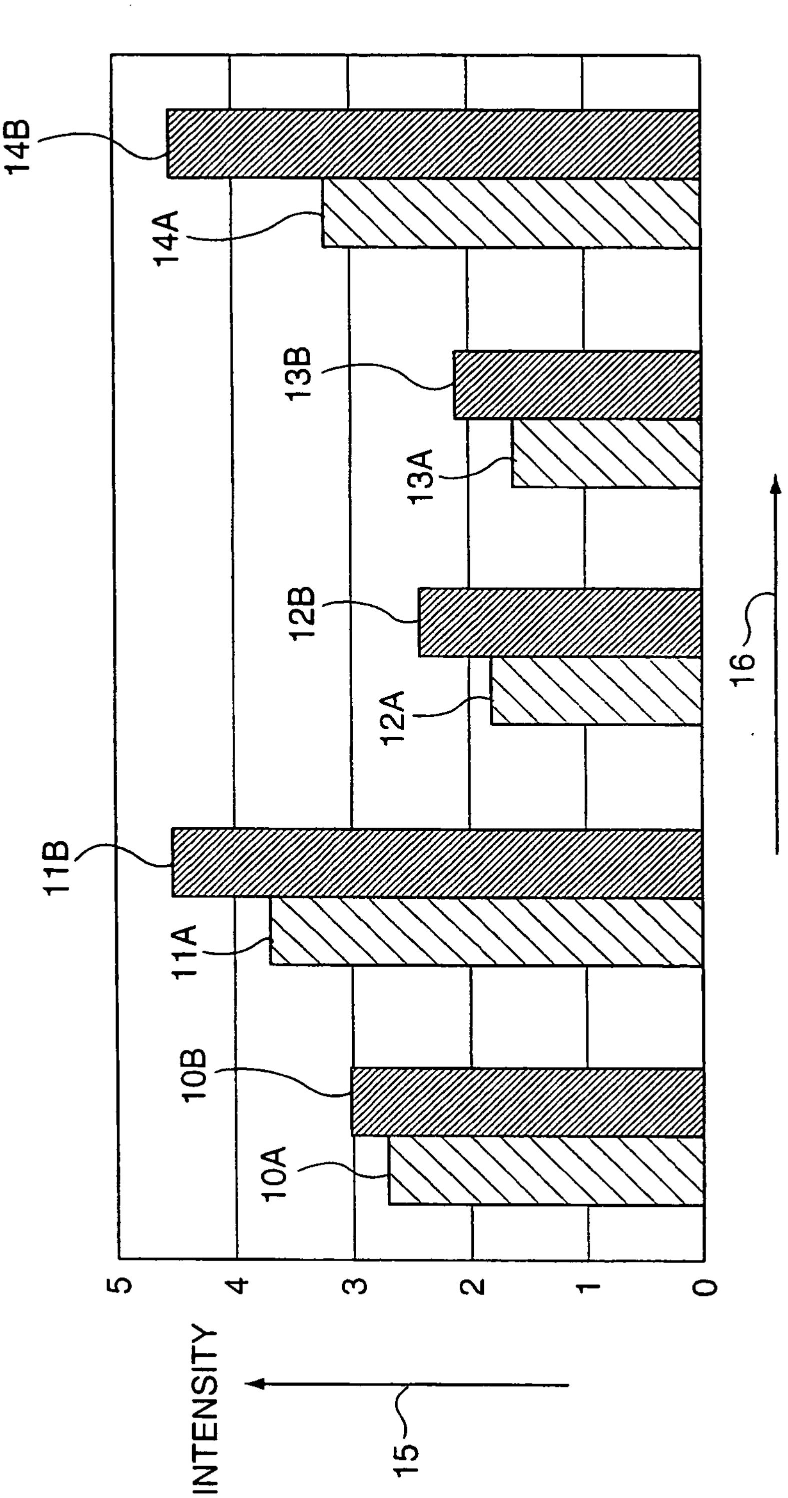


FIG. 1

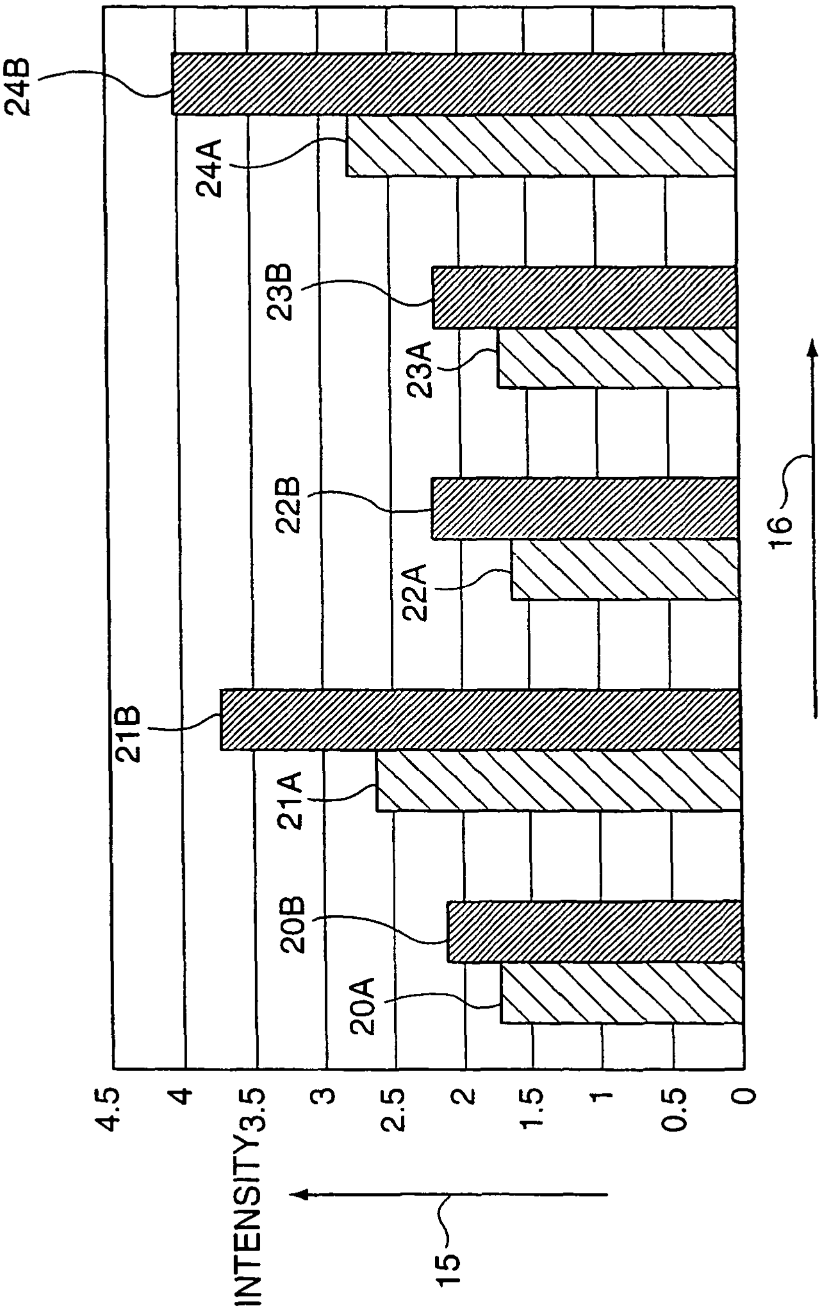
24B
24A
23B
23A
22B
22A
21B
21A
20B
20A
16
4.5
4
3.5
3
2.5
2
1.5
1
0.5
0
INTENSITY
15

FIG. 2

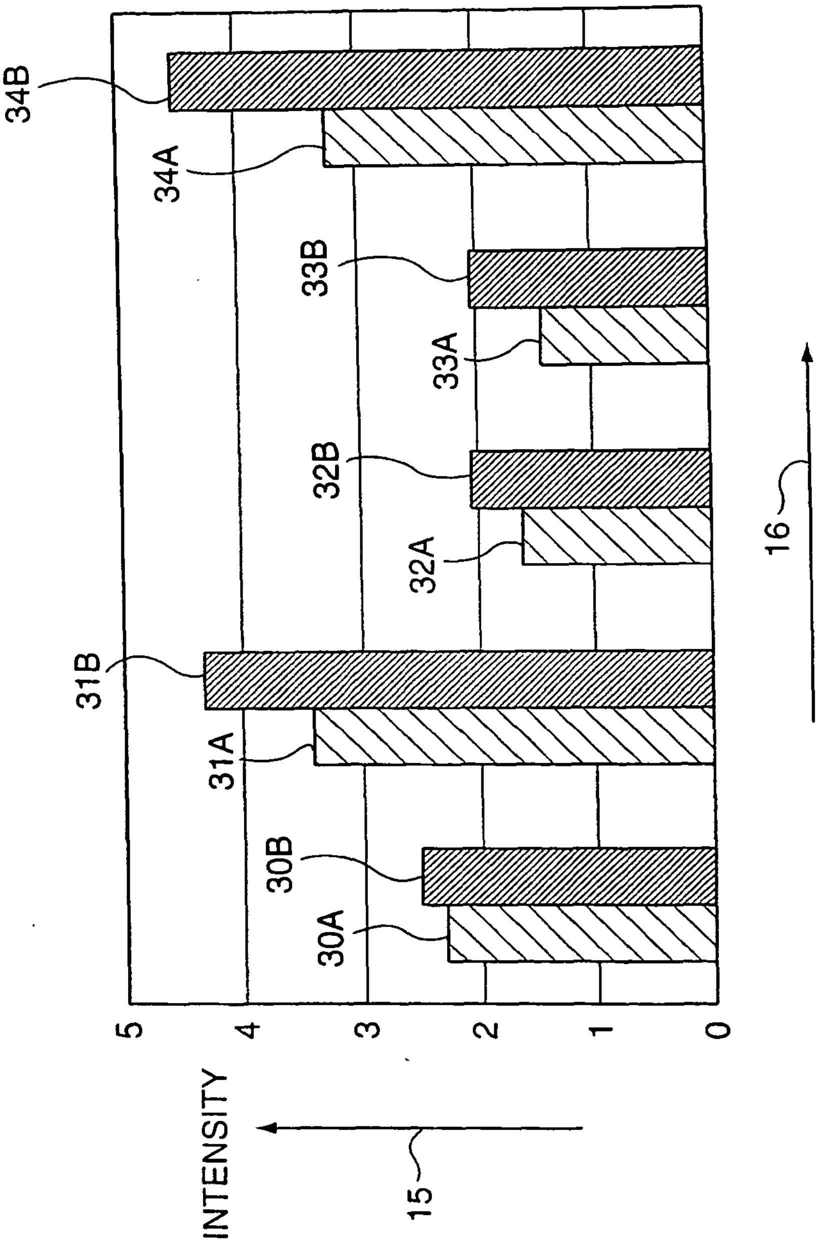


FIG. 3

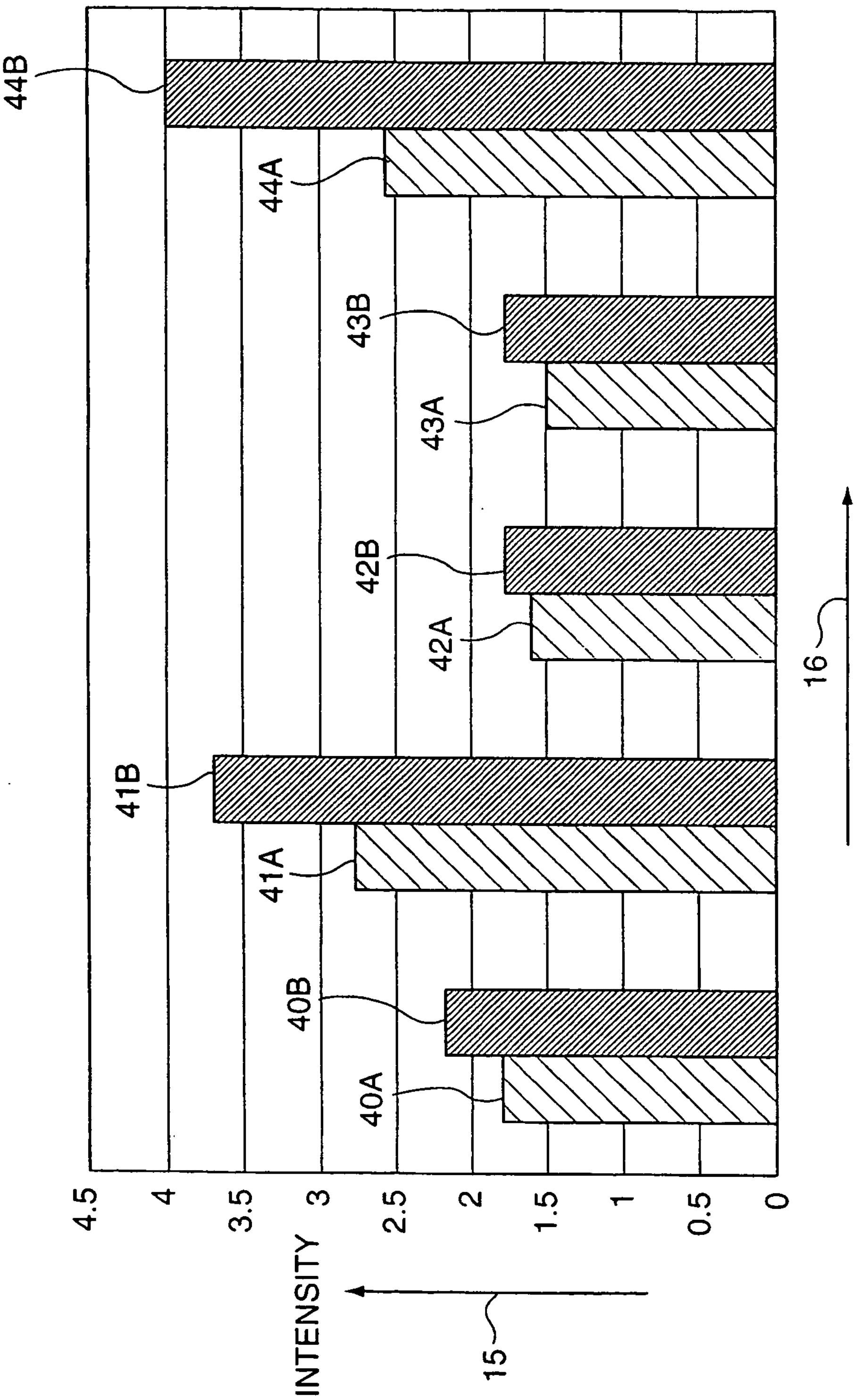
INTENSITY
15
4.5
4
3.5
3
2.5
2
1.5
1
0.5
0
40A
40B
41A
41B
42A
42B
43A
43B
44A
44B
16

FIG. 4

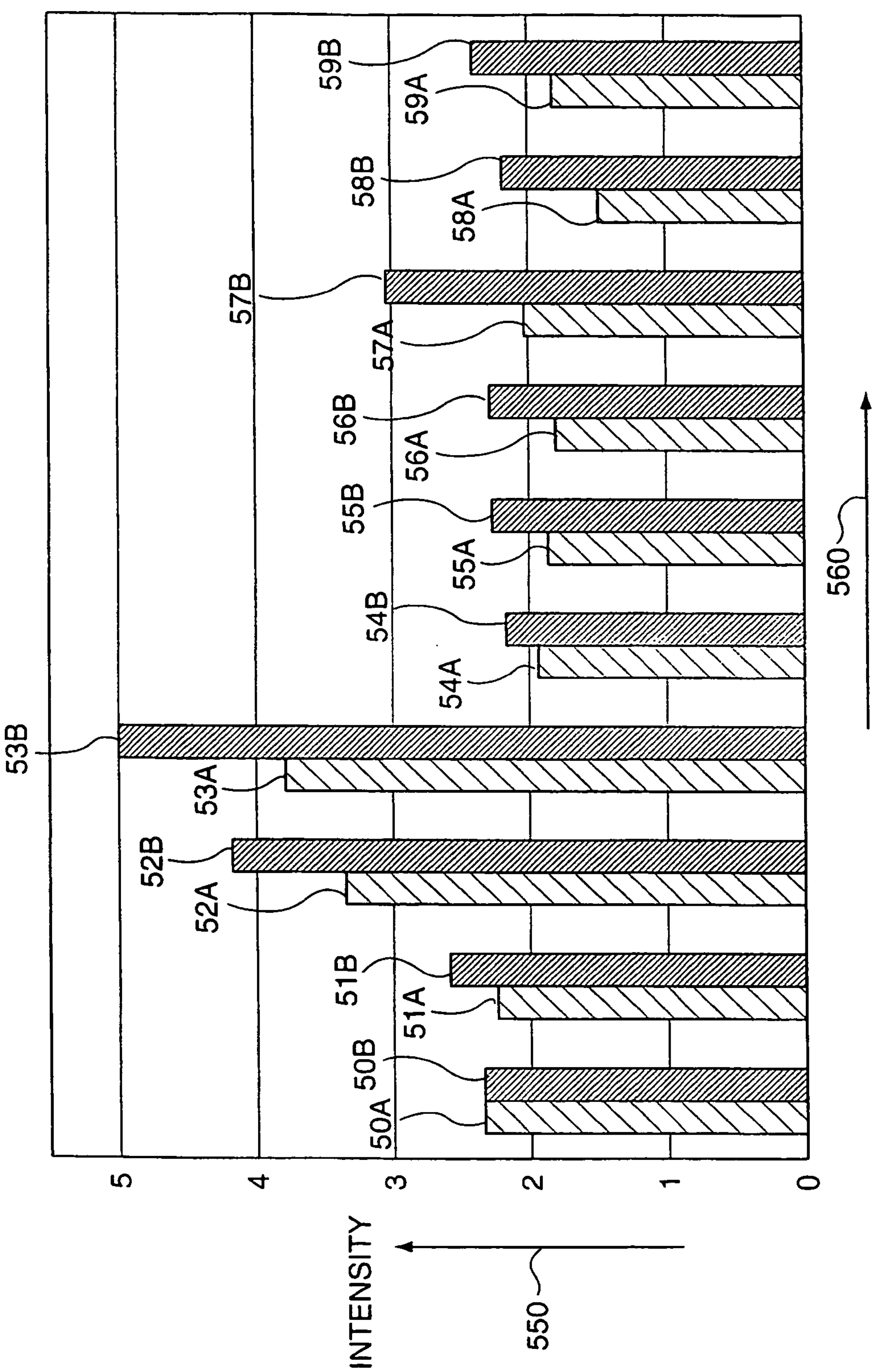
INTENSITY
550
0
1
2
3
4
5
50A
50B
51A
51B
52A
52B
53A
53B
54A
54B
55A
55B
56A
56B
57A
57B
58A
58B
59A
59B
560

FIG. 5

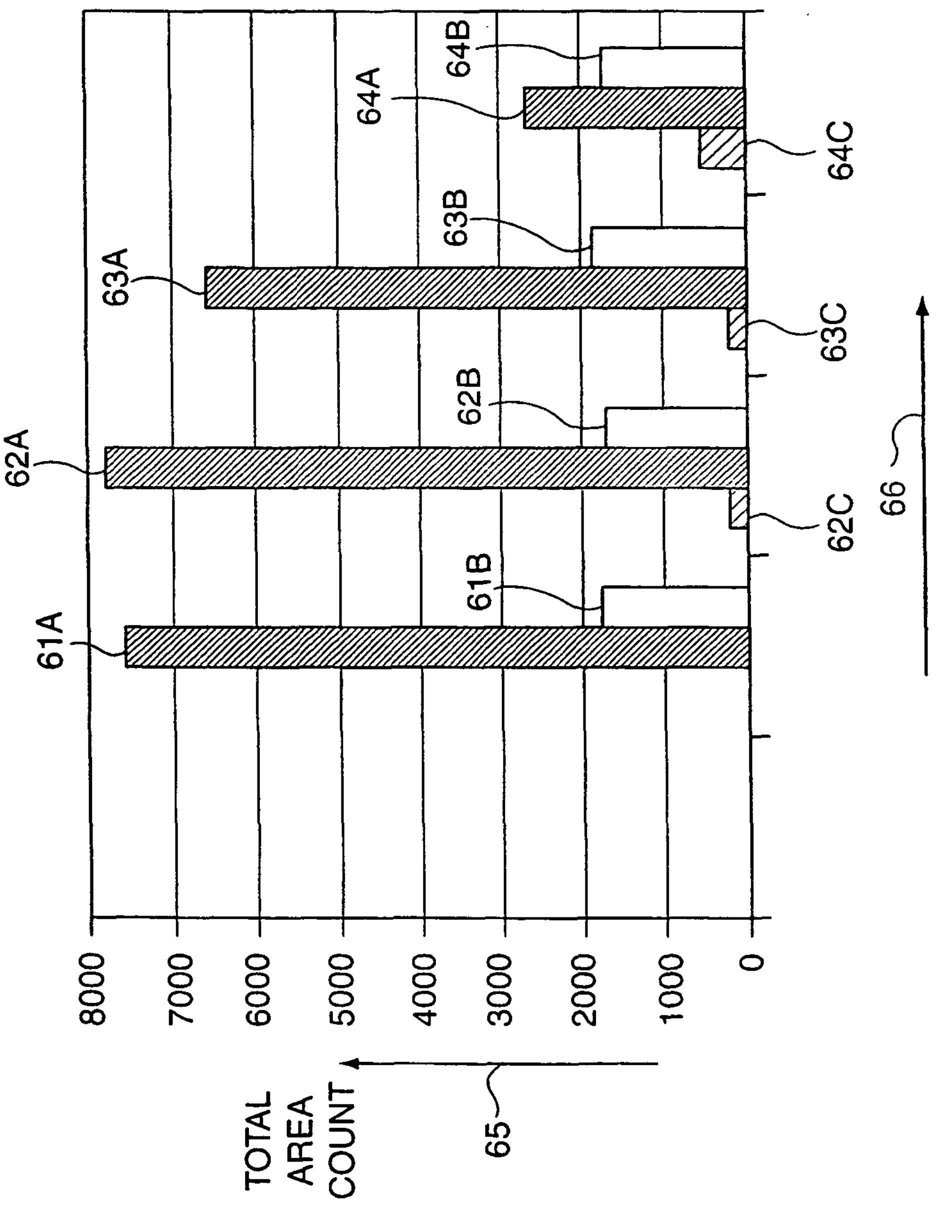
TOTAL AREA COUNT
65
8000
7000
6000
5000
4000
3000
2000
1000
0
61A
61B
62A
62B
62C
63A
63B
63C
64A
64B
64C
66

FIG. 6

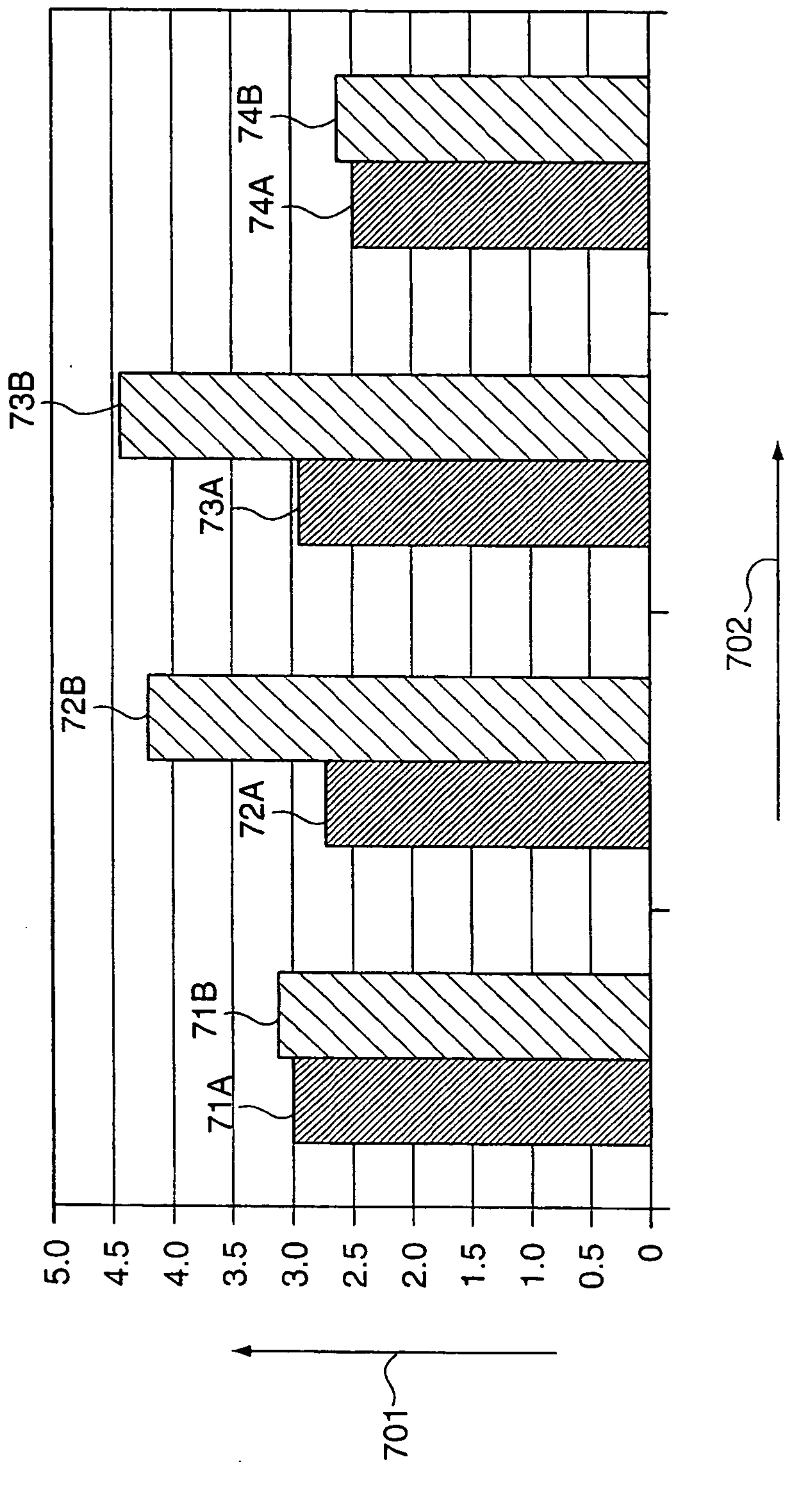
71A
71B
72A
72B
73A
73B
74A
74B
5.0
4.5
4.0
3.5
3.0
2.5
2.0
1.5
1.0
0.5
0
701
702

FIG. 7A

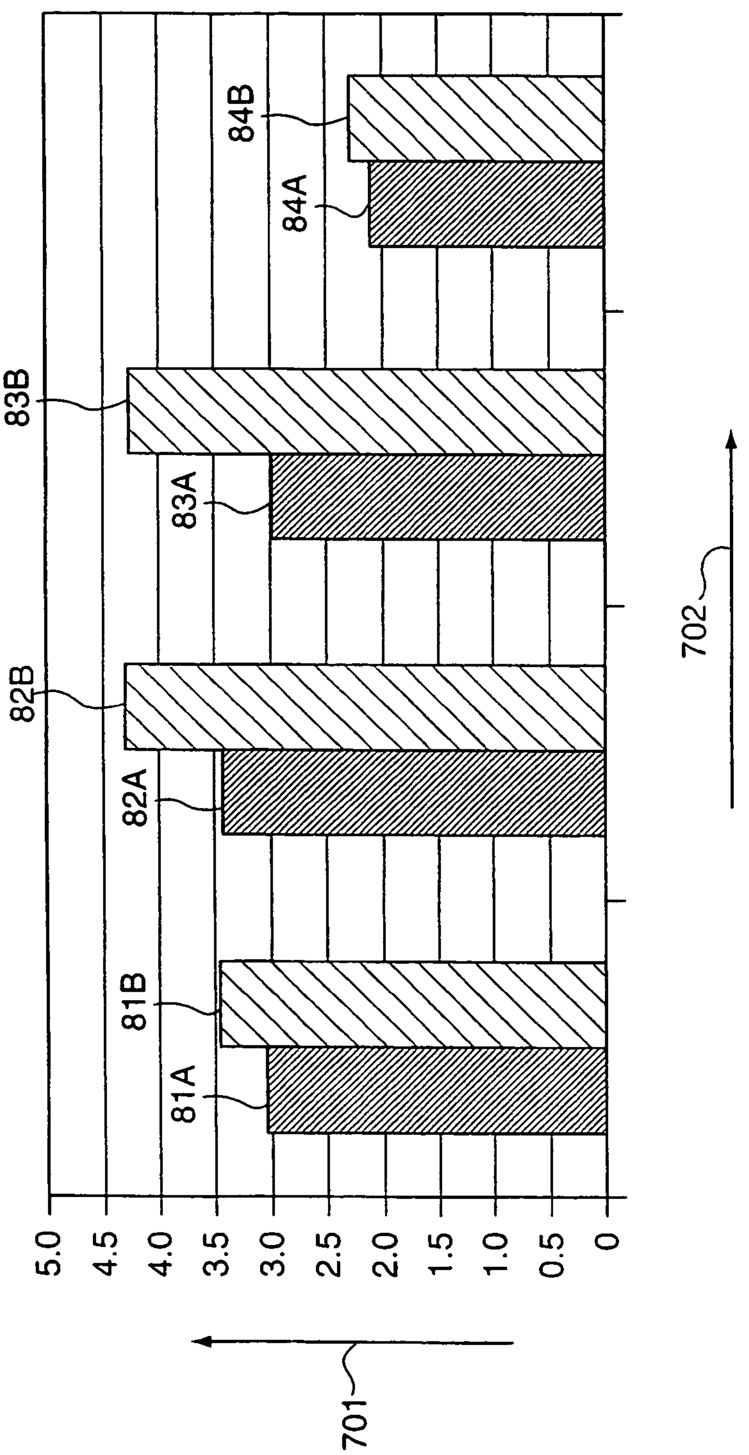
5.0
4.5
4.0
3.5
3.0
2.5
2.0
1.5
1.0
0.5
0
81A
81B
82A
82B
83A
83B
84A
84B
701
702

FIG. 7B

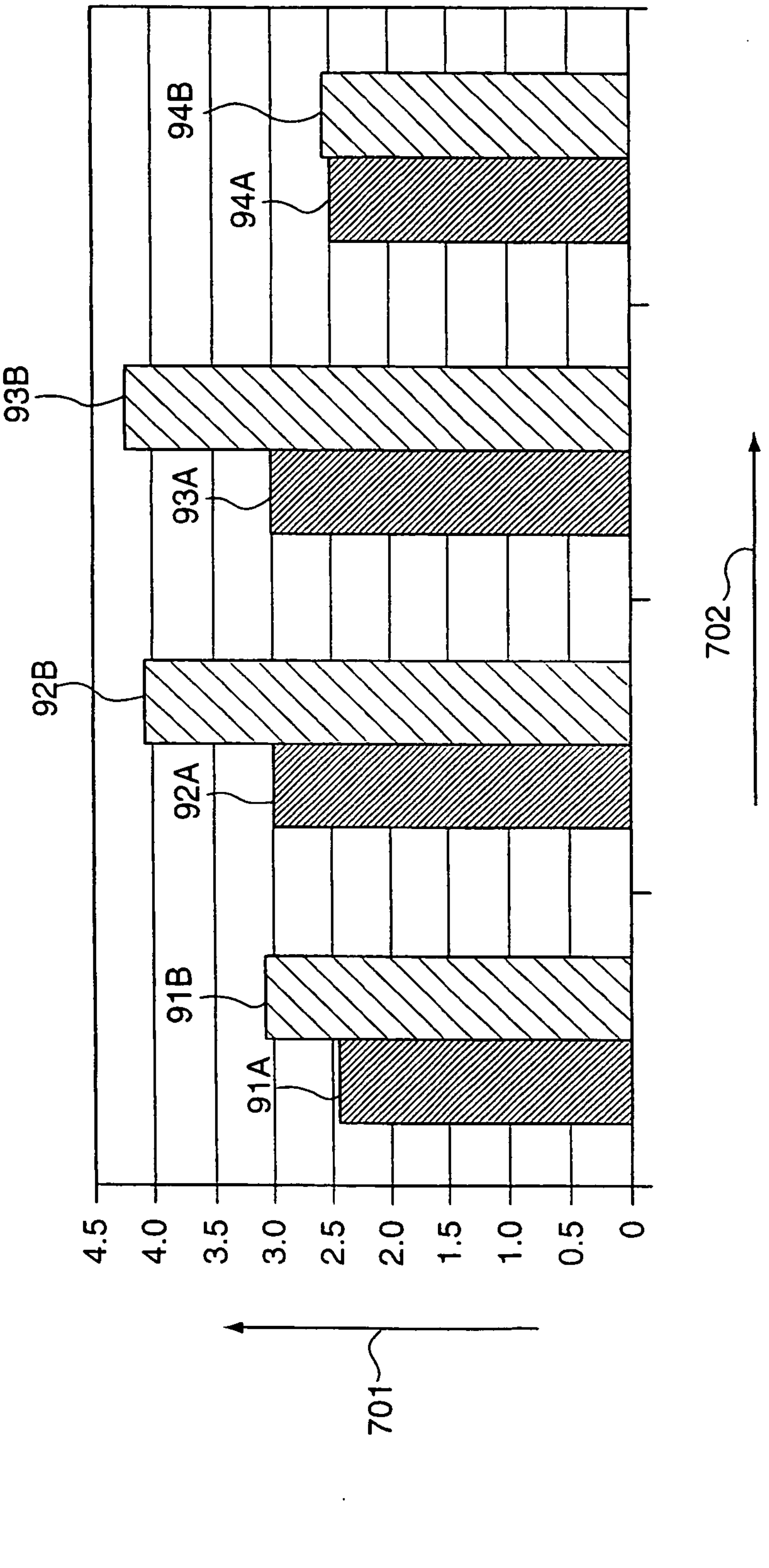


FIG. 7C

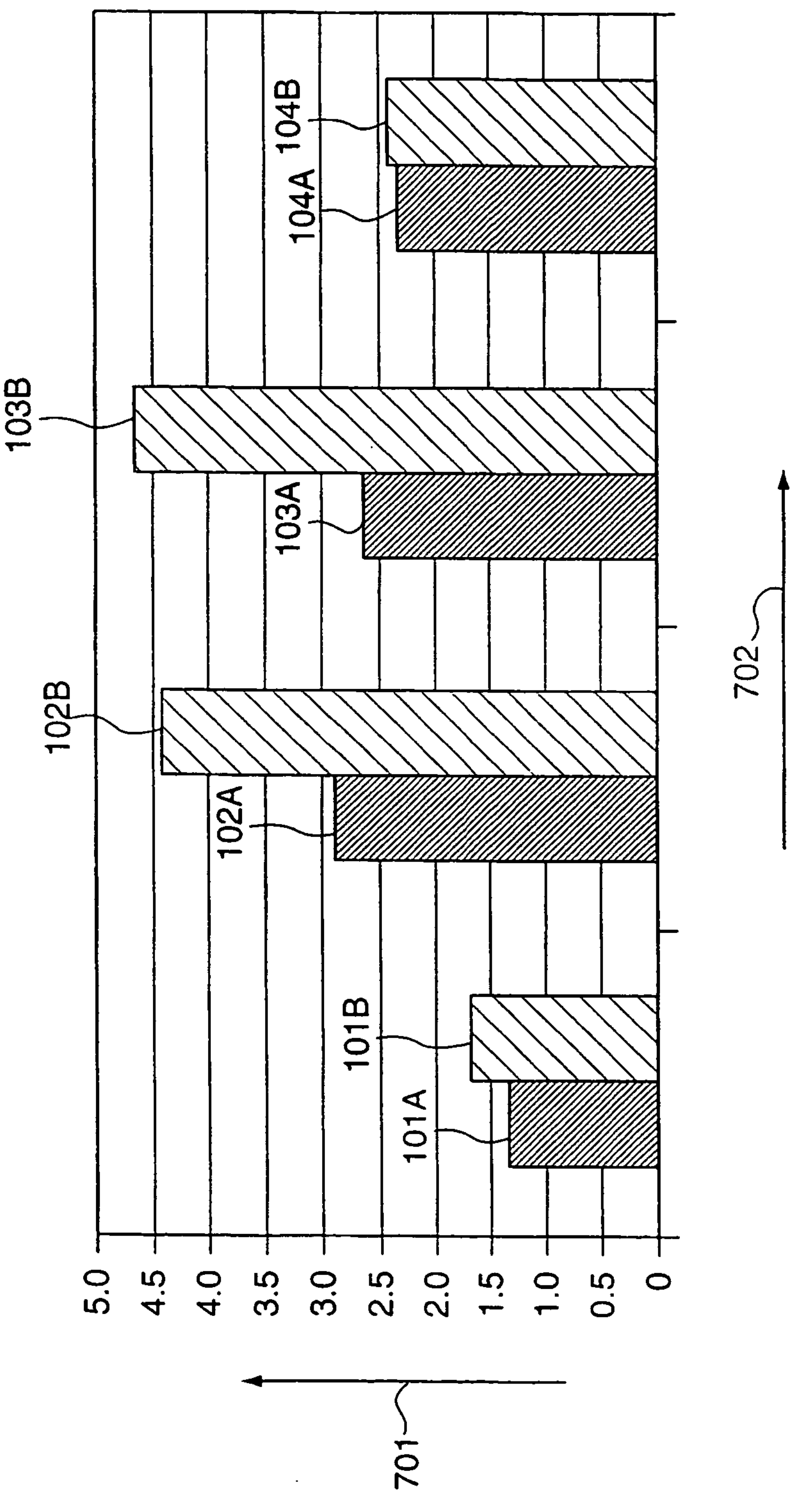
5.0
4.5
4.0
3.5
3.0
2.5
2.0
1.5
1.0
0.5
0
101A
101B
102A
102B
103A
103B
104A
104B
701
702

FIG. 7D

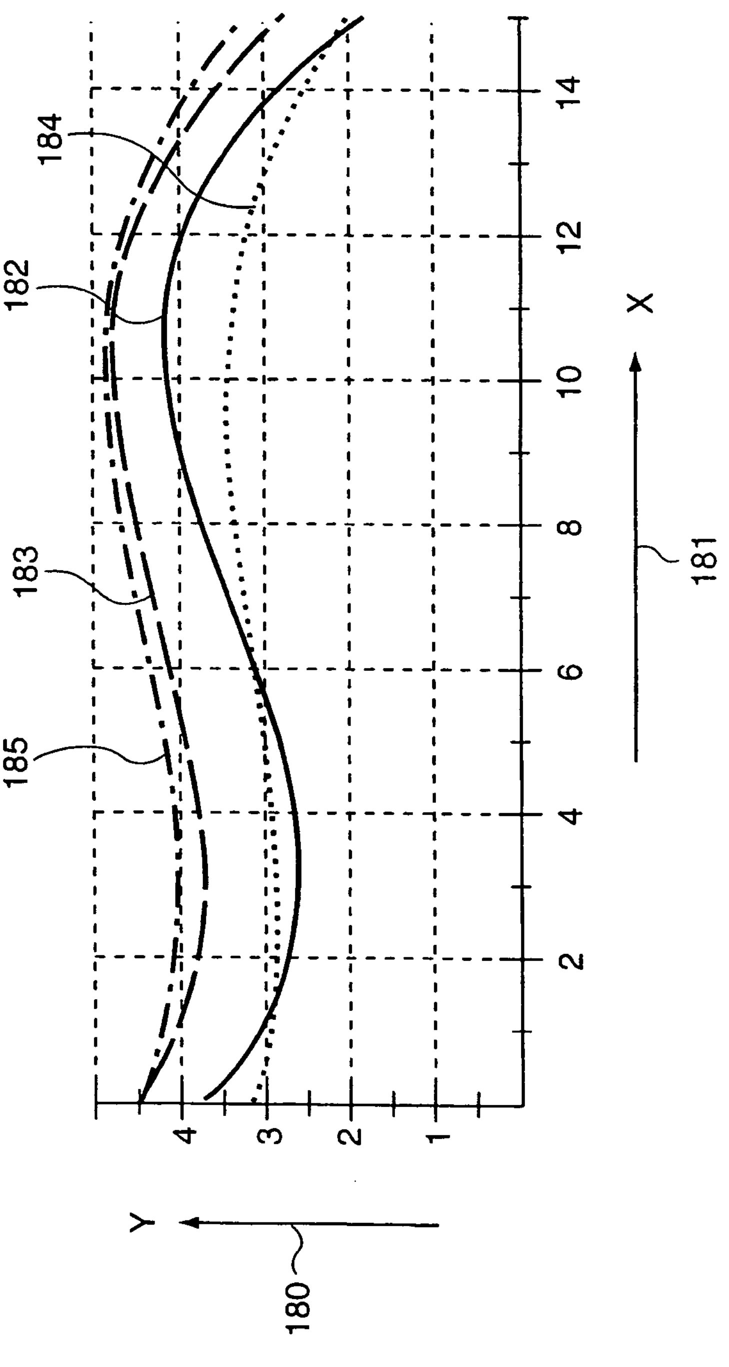


FIG. 8

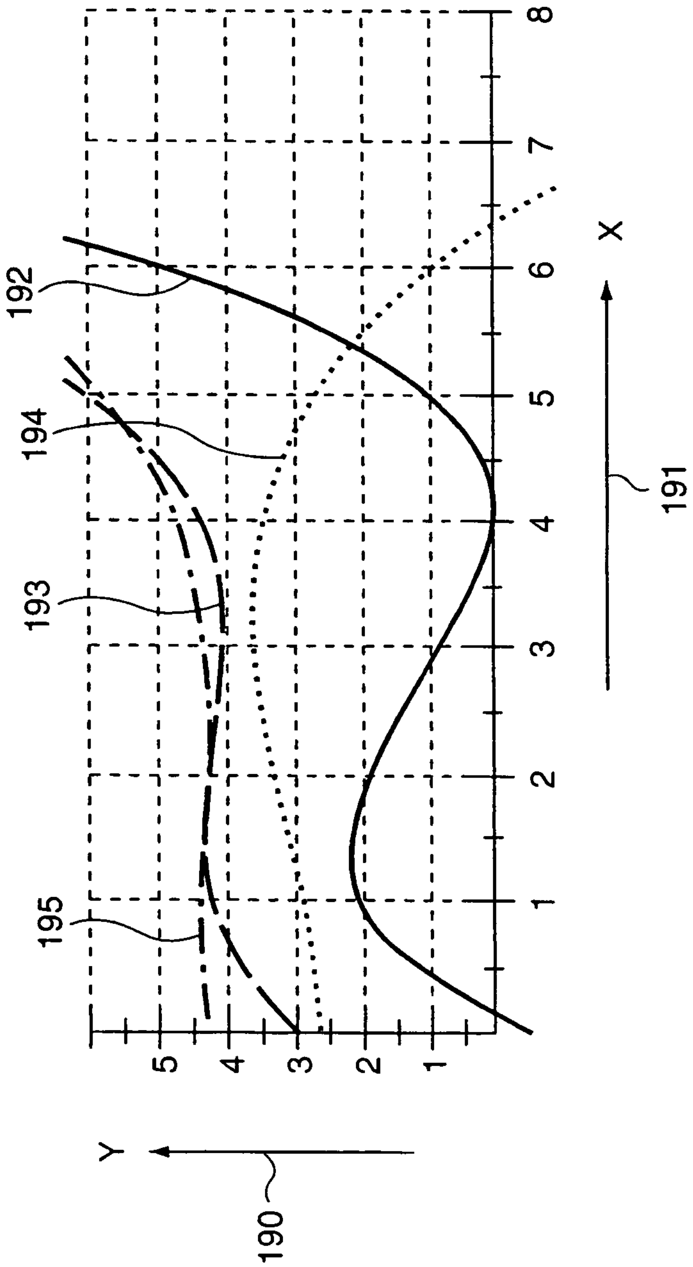


FIG. 9